# EUROPEAN PATENT SPECIFICATION

(11) **EP 0 451 194 B1**
(45) Date of publication and mention of the grant of the patent: **23.07.1997**
(21) Application number: 90901407.8
(22) Date of filing: 21.12.1989
(51) Int. Cl.: C07K 9/00, C07K 14/00, C12N 15/09, C12N 15/11, C12N 15/18, C12N 1/21, G01N 33/566, C12R 1/19

(54) **RECOMBINANT DNA MOLECULES, HOSTS, PROCESSES AND HUMAN SOMATOMEDIN CARRIER PROTEIN-LIKE POLYPEPTIDES**
REKOMBINANTE DNS-MOLEKÜLE, WIRTE UND DEM MENSCHLICHEN SOMATOMEDIN-TRÄGERPROTEIN ÄHNLICHE POLYPEPTIDE
MOLECULES D'ADN RECOMBINANT, HOTES, PROCEDES ET POLYPEPTIDES SIMILAIRES A DES PROTEINES PORTEUSES DE SOMATOMEDINE HUMAINE

(30) Priority: 22.12.1988 US 290250
(43) Date of publication of application: 16.10.1991
(73) Proprietor: CELTRIX PHARMACEUTICALS, INC., Santa Clara, CA 95052-8203 (US)
(72) Inventor: SPENCER, Emerald, Martin, San Francisco, CA 94112 (US); TALKINGTON-VERSER, Carol, San Rafael, CA 94903 (US)
(74) Representative: Armitage, Ian Michael
(86) International application number: US8905791
(87) International publication number: WO9006950

(56) References cited:
- WO-A-88/09818
- WO-A-89/08666
- WO-A-89/09268
- WO-A-89/09792
- US-A- 4 642 120
- US-A- 4 738 921
- US-A- 4 861 757
- US-A- 4 885 163
- Biochemical and Biophysical Research Communications, Published 30 September 1986, "Growth Hormone-Dependent Insulin-Like Growth Factor (IGF) Binding Protein from Human Plasma Differs from other Human IGF Binding Proteins", (Baxtere) Vol. 139, pages 1256-1261. See Abstract and whole publication.
- The Journal of Biological Chemistry, Published 05 July 1986, "Insulin-like Growth Factor-binding Protein from Human Plasma: Purification and Characterization", (MARTIN), Vol. 19, pages 8754-8760. See Abstract and whole publication.
- Journal of Clinical Endocrinology and Metabolism, Published 1984, "Immunoassay of a Sonatomedin-Binding Protein from Human Amniotic Fluid: Levels in Fetal, Neonatal, and Adult Sera", (DROP), Vol. 59, pages 908-915. See Abstract.
- Journal of Clinical Investigation, Published April 1985, (WILKINS), Vol. 75, pp. 1350-1358
- Journal of Clinical Endocrinology and Metabolism, Published 1980, "The Somatomedin C Binding Protein: Evidence for a Heterologous Subunit Structure", (FURLANETTO), Vol. 51, pages 12-19. See pages 15 and 17.
- Molecular and Cellular Endocrinology, Published 1984, "Somatomedin Carrier Proteins", (SMITH), Vol. 34, pages 83-89. See whole publication, especially p. 87.
- Biochemical and Biophysical Research Communications, Published 16 May 1985, "The Somatomedin-binding Protein Isolated from a Human Hepatoma Cell Line is Identical to the Human Amniotic Fluid Somatomedin-binding Protein", (POVOAE, Vol. 128, pages 1071-1078. See pages 1076 and 1077.
- Journal of Endocrinology, Published September 1988, "The Role of Growth Hormone in Diabetes Mellitus", (HOLLY), Vol. 118, pages 353-364. See whole publication.
- Journal of Clinical Investigation, Published December 1986, (BAXTER), Vol. 78, pp. 1504-1512
- Journal of Clinical Endocrinology and Metabolism, Published 1985, "Antibody Against Acid-stable Insulin-like Growth Factor Binding Protein Detects 150,000 Mol Wt Growth Hormone-Dependent Complex in Human Plasma", (MARTIN), Vol. 61, pages 799-801. See p. 801.
- Journal of Clinical Endocrinology and Metabolism, Published December 1984, "Serum Levels of Insulin-like Growth Factor (IGF) and IGF Binding Protein in Constitutionally Tall Children and Adolescents", (GOURMELEN), Vol. 59, pages 1197-1203. See the Abstract.
- Journal of Biological Chemistry, Published July 25 1987, "Insulin-like Growth Factor I Receptors in Retinal Rod Outer Segments", (ZICK), Vol. 262, pages 10259-10264. See the Abstract.
- Journal of Clinical Endocrinology and Metabolism, Published July 1982, (SCHALCH), Vol. 55, pp.49-55
- Journal of Clinical Endocrinology and Metabolism, Published August 1988, "Serum Bone Gla-protein as a Marker of Bone Growth in Children and Adolescents: Correlation with Age, Height, Serum Insulin-like Growth Factor I, and Serum Testosterone", (JOHANSEN), Vol. 67, pages 273-278. See the Abstract.
- BIOCHEMICAL AND BIOPHISICAL RESEARCH COMM. vol. 152, no. 3, May 1988, pp. 1289-1297 New York , US; M.T. BREWER et al.
- THE EMBO JOURNAL vol. 7, no. 8, August 1988, pp. 2417-2423, Oxford, GB; A. BRINKMAN et al.
- ENDOCRINOLOGY vol. 111, no. 3, September 1982, pp. 801-805, Baltimore, US; D.H. MORRIS et al.:
- THE JOURNAL OF CLINICAL ENDOCRINOLOGY & CLINICAL METABOLISM vol. 51, no. 1, July 1980, pp. 12-19, Philadelphia, US; R.W. FURLANETTO:
- THE JOURNAL OF CLINICAL ENDOCRINOLOGY & METABOLISM vol. 61, no.4, October 1985, pp. 799-801, Philadelphia, US; J.L. MARTIN et al.:
- BIOCHEMICAL AND BIOPHYSICAL RESEARCH COMMUNICATIONS vol. 156, no. 3, November 1988, pp. 1187-1194, New York, US; J. ZAPF et al.:
- JOURNAL OF CLINICAL ENDOCRINOLOGY AND METABOLISM vol. 59, no. 5, 1984, pp. 908-915, Philadelphia, US; S.L.S. DROP et al.:

## Description

This invention relates to human somatomedin carrier protein subunits and to processes for producing them. More particularly, this invention relates to carrier protein subunits that bind to human somatomedin-like polypeptides, also known as insulin-like growth factors. In addition, this invention relates to essentially pure human somatomedin carrier protein subunits. The carrier protein subunits and methods of this invention may be used in a variety of therapeutic, diagnostic or other useful applications.

Also described are DNA molecules encoding human somatomedin carrier protein-like polypeptides, recombinant DNA molecules, hosts transformed with such molecules, processes for producing human somatomedin carrier protein-like polypeptides, and human somatomedin carrier protein-like polypeptides produced using those molecules hosts and processes. More particularly, the above-mentioned DNA molecules can be expressed in appropriate hosts. The recombinant DNA molecules contain DNA molecules that code for polypeptides which have a biological activity of the human carrier protein. As will be appreciated from the disclosure to follow, the DNA molecules, recombinant DNA molecules, hosts, and processes may be used in the production of polypeptides useful in a variety of therapeutic, diagnostic, and other useful applications.

Somatomedins (also sometimes referred to as "SMs") are hormones having useful biological properties. SMs are polypeptides having a molecular weight of approximately 7,500 daltons. SMs (a) mediate the growth-promoting effects of growth hormone (also sometimes referred to as "GH"), (b) have weak insulin-like activity (and for that reason are also called "insulin-like growth factors" or "IGFs"), (c) are mitogenic for a variety of skeletal and other tissues and (d) are transported in plasma bound to a large carrier protein. There are two SM compositions in humans. SM-C is a basic polypeptide and is sometimes referred to as SM-C. SM-C mediates the growth promoting actions of GH after birth. SM-A is a mixture primarily of a polypeptide known as IGF-II and variable amounts of a modified form of SM-C. Spencer, E.M., et al., "The Identity Of Human Insulin-like Growth Factors I and II With Somatomedins C and A With Rat SM I and II" in Insulin-like Growth Factors/Somatomedins; ed. Spencer, E.M. (Walter de Gruyter 1983). IGF-II is less GH dependent and may have a role in fetal growth.

SMs may be useful in vivo to stimulate bone formation (for example, in treatment of osteoporosis), wound healing, and the growth of animals and GH-deficient humans. Serum levels of SM-C are measured to diagnose acromegaly, pituitary gigantism, GH deficiency, and other growth related conditions. Spencer, E.M., "Somatomedins" in Basic and Clinical Endocrinology, eds. Green-span F. S. and Forsham, P. H. (1986), p. 89. Appleton-Century-Crofts. SMs are also employed to stimulate in vitro the proliferation of a variety of cells in tissue culture and, therefore, are useful in the study of the regulation of normal and abnormal cell growth. SMs produced by certain breast and kidney cancer cells may stimulate the proliferation of both the cancer cells and the vascular and fibrous tissues required to support the growth of the cancer tissues. Spencer, E. M. et al., "Possible Auto-stimulation of Human Mammary Carcinoma Growth by Somatomedins," Annals of the N.Y. Acad. Sci., 464, p. 448 (1986); Huff, K.K., et al., "Secretion of Insulin-like Growth Factor-I-related Protein by Human Breast Cancer Cells," Cancer Research 46, pp. 4613-4619 (1986). Blocking the action of SMs may be useful to control the growth of these cancers.

Human SMs appear to be transported and regulated in vivo by other proteins. Hintz, R. L. et al., "Demonstration of Specific Plasma Protein Binding Sites For Somatomedin," J. Clin. Endocrinol. Metab. 45, p. 988 (1977). These proteins appear to bind to the SMs and regulate the biological activity of the SMs in vivo. Gel filtration of human serum at neutral pH has shown that 95% of the immunoreactive SM-C activity, and probably IGF-II activity, elutes at about 150,000 to 160,000 daltons (150-160 kilodaltons or "kDa") with a minor amount in the range of 35-50 kDa. Only a very small amount of immunoreactive activity elutes at 7.5 kDa, where free SMs should appear. Smith. G. L., Molecular and Cellular Endocrinology 34, p. 83-89 (1984). This indicates that SMs are complexed with larger proteins in plasma.

At least two different classes of proteins or protein complexes in human plasma have been reported to bind SMs. Drop, S. L. et al.. "Immunoassay Of A Somatomedin-binding Protein From Human Amniotic Fluid; Levels In Fetal, Neonatal, And Adult Sera," J. Clin. Endocrinol. Metab. 59, p. 908 (1984); Wilkins, J. R. et al., "Affinity-labeled Plasma Somatomedin-C+/Insulin-like Growth Factor I Binding Proteins." J. Clin. Invest. 75, p. 1350 (1985). This description refers to one class of those native proteins or protein complexes as the SM "Carrier Protein" for its function appears to be the transport of SMs. This term is not intended to indicate that the carrier protein is a single protein. There may be more than one carrier protein and it may be a protein complex. This description refers to the other class as the "Amniotic Fluid Binding Protein" or "AFBP." There may be more than one AFBP. It is also possible that additional classes of proteins or protein complexes that bind SMs will be discovered.

Carrier protein activity, like SM-C activity, is GH-dependent, being low in persons with GH deficiency and elevated in patients with GH-producing tumors, a condition known as acromegaly. White, R. M., et al., "The Growth Hormone Dependence Of Somatomedin-binding Protein In Human Serum," J. Clin Endocrinol Metab. 53, p. 49 (1981). The carrier protein displays biological properties indicative of potentially valuable uses. In vivo, when SMs bind to carrier protein, the half-life of the SMs is reported to increase from approximately one hour to up to about 24 hours depending on the animal species tested (Cohen. K. L. et al., "The Serum Half-life Of Somatomedin Activity: Evidence For Growth Hormone Dependence," Acta Endocrinol. 83, p. 243 (1976)), and the SMs are rendered inactive until released. Studies in other model systems suggest that impure preparations containing the carrier protein (a) abolish the metabolic action of the SMs on the perfused rat heart (Meuli C., et al., "NSILA-carrier Protein Abolishes The Action Of Nonsuppressible Insulin-like Activity (NSILA-s) On Perfused Rat Heart," Diabetologia 14, p. 255 (1978)), (b) inhibit the mitogenic effect of the SMs on cells in culture (Knauer, D. J., Proc. Natl. Acad. Sci. U.S.A., 77, pp. 7252-7256 (1980) and Kuffer, A. D., et al., "Partial Purification Of A Specific Inhibitor Of The Insulin-like Growth Factors By Reversed Phase High-performance Liquid Chromatography," J. of Chromatography, 336, pp. 87-92 (1984) and (c) block the insulin-like activity of SMs on rat adipose tissue (Zapf, J., et al., "Inhibition Of The Action Of Nonsuppressible Insulin-like Activity On Isolated Rat Rat Cells By Binding To Its Carrier Protein," J. Clin Invest. 63, p. 1077 (1979). Partially pure preparations of the carrier protein have been used with radiolabeled SMs in research to conduct competitive binding assays for measuring SMs. Moses, A. C., et al., Endocrinology 104, p. 536 (1979).

Because of their valuable biological properties, there have been many efforts to isolate and characterize the carrier protein or the subunits of the carrier protein responsible for that activity. Previous attempts to isolate and characterize in pure form the carrier protein or its active subunits have failed. This is due in part to the low concentration of carrier protein in plasma. A successful purification procedure also had to solve the problems of loss of activity because of enzymatic digestion and instability of the carrier protein, especially to changes in pH. Purification of the carrier protein subunits is further complicated by the presence in plasma of the AFBP, which also binds to somatomedins.

The carrier protein is a glycoprotein. In serum at neutral pH, it is bound with SMs and the complex has a molecular weight of about 150-160 kDa when measured by gel filtration. The molecular weight of the carrier protein complex at neutral pH has also been determined by other methods to be about 125 kDa. Gel filtration chromatography of serum or plasma under acid conditions has been reported to separate bound SMs from the carrier protein and to give rise to a unit of the carrier protein that has a molecular weight of about 40-50 kDa. That unit also binds to somatomedins. Hintz, R. L., et al., "Demonstration Of Specific Plasma Protein Binding Sites For Somatomedin," J. Clin. Endocrinol. Metab. 45, p. 988 (1977). Since the 40-50 kDa acid-stable unit cannot be induced to reform the 150-160 kDa carrier protein complex, others have suggested that the carrier protein may also be composed in part of an acid-labile unit that does not itself bind to somatomedins. Moses, A. C., et al., Endocrinology 104, p. 536 (1979). Furlanetto reported treating serum with a 35-55% ammonium sulfate solution, isolating the precipitate, dissolving the precipitate in 0.05 M Tris, pH 8.20 and chromatographing on DEAE Sephadex A-50 with Tris buffers. Furlanetto, R. W., "The Somatomedin C Binding Protein: Evidence For A Heterologous Subunit Structure," J. Clin, Endocrinol Metab. 51, p. 12 (1980). Furlanetto did not disclose any further purification. Rather, Furlanetto conducted experiments with various fractions to confirm his view that the somatomedin-C binding activity in serum is composed of at least two units, one has a Stokes radius of 36 A° and binds SM-C (the so-called acid stable unit) and the other has Stokes' radius of 30-38 A° and does not bind SM-C (the so-called acid labile unit)).

Wilkins identified, by affinity labeling', plasma proteins that complexed with SM-C. Wilkins, J. R., et al., "Affinity-labeled Plasma Somatomedin-C/ Insulin-like Growth Factor I Binding Proteins," J. Clin. Invest., 75, p. 1350 (1985). ¹²⁵I-SM-C was covalently cross-linked to proteins that bound SM-C in whole plasma and in Sephadex G-200 fractions of plasma. Following sodium dodecylsulfate polyacrylamide gel electrophoresis and autoradiography, the AFBP was identified in addition to species of about 160, 110, 80, 50 and 25 kDa. Wilkins et al. hypothesized that the 160 kDa carrier protein complex consisted of 6 approximately 25 kDa (24-28 kDa) subunit complexes, each composed of the subunit plus SM-C. However, Wilkins et al., did not report isolation or purification of this 25 kDa subunit. Another worker proposed, but did not establish, a slightly larger subunit structure. Daughaday, W. H., et al., "Characterization Of Somatomedin Binding in Human Serum By Ultracentrifugation And Gel Filtration," J. Clin. Endocrinol. Metab. 55, p. 916 (1982).

Several workers have reported unsuccessful attempts to isolate the acid-stable 40-50 kDa carrier protein unit from human plasma. Draznin et al., reported a material containing only 1% SM binding activity and did not disclose whether this material originated from carrier protein or AFBP. Draznin, B., et al., in "Somatomedins and Growth," eds. G. Giordano et al. (Academic Press 1979) pp. 149-160. Fryklund et al., fractionated fresh frozen human plasma by polyethylene glycol precipitation, carboxymethyl-Sephadex chromatography, and gel filtration. Fryklund, L., et al., in Hormones and Cell Culture, eds G. H. Sato et al. (Cold Spring Harbor Laboratory 1979) pp. 49-59. Fryklund et al., proposed that the carrier protein consisted of 2 dissimilar chains of 35 and 45 kDa. Fryklund et al., disclosed that glycine was released by N-terminal molecule analysis, but did not identify from which chain it originated or whether both ended in glycine. The reported binding activity of the Fryklund et al. preparation was very low and purity was not reported. Fryklund et al. did not establish whether the carrier protein or the AFBP was present in their preparation.

None of the above studies disclose a class of human carrier protein subunits capable of binding somatomedin-like polypeptides. In addition, none of these studies disclose any subunits of the carrier protein capable of binding SMs and purified to homogeneity. Purity is required to establish that the carrier protein has been isolated instead of the AFBP or a contaminant and to study biologic activity. An impure preparation may contain enzymes, causing the product to be unstable, and easily degraded or denatured. Impure preparations also cannot be used in animals and humans, because many impurities present in original serum or produced as a result of the purification procedures, are antigenic and could produce unwanted biologic effects. For example, human use in osteoporosis requires removal of all contaminants, which may be antigenic or have adverse biologic effects.

Other workers have isolated a different protein capable of binding SMs and obtained from mid-gestational amniotic fluid of humans, the amniotic fluid binding protein or "AFBP." The AFBP is not the carrier protein or a subunit of the carrier protein. Wilkins, J. R. et al., "Affinity-labeled Plasma Somatomedin-C/Insulin-like Growth Factor I Binding Proteins," J. Clin. Invest. 75, p. 1350 (1985). The AFBP (a) is smaller than the so-called acid-stable unit of the carrier protein, having a molecular weight in the range 32-40 kDa, (b) is not glycosylated, (c) differs from the carrier protein subunits of this invention in its reported N-terminal molecule (Povoa, G. et al., "Isolation And Characterization of A Somatomedin-binding Protein From Mid-term Human Amniotic Fluid," Eur. J. Biochem. 144, pp. 199-204 (1984)), and (d) has different immunologic properties. Drop, S. L. S. et al., "Immunoassay of A Somatomedin-Binding Protein From Human Amniotic Fluid: Levels In Fetal, Neonatal and Adult Sera,"J. Clin. Endocrinol. Metab. 59, p. 908 (1984); Martin, J. L. et al., supra, J. Clin. Endocrinol. Metab. 61, pp. 799-801 (1985). Antisera to the AFBP do not cross-react with the 150 kDa carrier protein or its acid-stable unit. Drop et al. reported that the AFBP levels determined by radioimmunoassay (RIA) were found to decrease during infancy and childhood - the inverse of the carrier protein - and also, unlike the carrier protein, to have a significant diurnal variation. Enberg also isolated the AFBP from adult human plasma by four chromatographic steps: CM-Affigel blue, hydroxylapatite, fast protein liquid chromatography gel permeation and high performance liquid chromatography ("HPLC") hydroxylapatite. Enberg, G., "Purification of A High Molecular Weight Somatomedin Binding Protein From Human Plasma," Biochem. and Biophy. Res. Commun., 135, pp. 178-82 (1986). Enberg reported a "possible" N-terminal molecule, Ala-Pro-Trp-, demonstrating that the AFBP was isolated, not the 150 kDa carrier protein as Enberg erroneously concluded.

Proteins that bind SMs have also been identified in cell culture extracts (e.g., Adams, S. O., et al. Endocrinology 115, pp. 520-526 (1984)). Thus far, the carrier protein has not been isolated. Spencer first showed that primary cultures of liver cells produced a protein that complexes with SMs. Spencer, E. M, "The Use Of Cultured Rat Hepatocytes To Study The Synthesis Of Somatomedin And Its Binding Protein," FEBS Letters, 99, p. 157, (1979). Subsequently, several cell types, normal and abnormal, have been found to synthesize a protein that complexes with SMs. Cultured Buffalo rat liver tumor cells (BRL 3A) produce a 33 kDa SM binding protein that differs from the carrier protein by antibody reactivity, N-terminal amino acid molecule, and absence of glycosylation. Lyons R. M. et al., Characterization of Multiplication-Stimulatory Activity "MSA" Carrier Protein," Molecular and Cellular Endocrinol. 45, pp. 263-70 (1986). Mottola. C. et al., J. of Biol. Chem., 261, pp. 1180-88 (1986). Romanus et al. reported that antibodies to this binding protein cross-reacted with a protein present in fetal serum but not adult rat serum. Romanus, J. A. et al., "Insulin-like Growth Factor Carrier Proteins In Neonatal And Adult Rat Serum Are Immunologically Different: Demonstration Using A New Radioimmunoassay For The Carrier Protein From BRL-3A Rat Liver Cells," Endocrinology, 118, p. 1743 (1986). The BRL-3A binding protein may be the rodent equivalent of the AFBP, but the N-terminal molecule data show no similarity between the two molecules.

Many proteins and polypeptides have been produced by use of recombinant DNA techniques. There is no published report of production of carrier protein-like polypeptides in this manner. There are numerous obstacles to using the techniques of recombinant DNA technology to clone and express a carrier protein-like polypeptide gene. Obtaining a gene encoding a carrier protein-like polypeptide is difficult for a variety of reasons. Prior to the invention, the protein sequences of the carrier protein and the carrier protein subunits were unknown and, therefore, DNA molecules that would code for the subunits were unknown. No human tissue source was established. Fibroblasts had been shown to produce small amounts of a large uncharacterized SM binding protein (Adams, S. O., et al. Endocrinology 115, pp. 520-526 (1984)). While liver is the major source of somatomedins, it had never been shown to produce the carrier protein. In addition, the liver is difficult to use to isolate mRNA, due to ribonucleases. The quantities of carrier protein in serum are very low. Thus, mRNA might be rare. The genome including a DNA molecule coding for the carrier protein may contain intervening sequences. For these and other reasons, many pitfalls faced the conventional approach to attempt to isolate a gene encoding a carrier protein-like polypeptide -- namely, identifying a source of mRNA containing large amounts of the desired molecule, creating a library of cDNA from that mRNA, screening the library with oligonucleotide probes designed to hybridize with cDNA having the desired molecule, and isolating or assembling a gene from those cDNA molecules.

In this description, the following terms are employed:

Somatomedin-like - A polypeptide displaying the biological activities of one of the human SMs or insulin-like growth factors, including but not limited to SM-C, SM-A, IGF-I and IGF-II. That polypeptide may have amino acids in addition to those of native human SMs or it may not include all the amino acids of native human SMs.

Carrier Protein - A glycoprotein or complex of glycoproteins in human plasma, displaying the ability to regulate the biological activity of the human SMs in vivo by a process involving binding of the SM-like polypeptides, being growth hormone dependent, and exhibiting an apparent molecular weight of about 125,000-160,000 daltons in physiological pH conditions when complexed with SMs. The carrier protein may also be polymorphic. For example, cells of different individuals may produce carrier protein species which are physiologically similar, but structurally slightly different from the prototype.

Subunit - A polypeptide fragment, part, or component of a larger protein unit. The term subunit is not confined to its customary meaning of a discrete polypeptide chain bound by covalent or any other types of bonds to another discrete polypeptide chain.

Carrier Protein Subunits - A class of subunits of the carrier protein.

Polypeptide - A linear chain of amino acids connected by peptide bonds. A polypeptide may also contain one or more disulfide bonds between cystines of the same amino acid chain.

Carrier Protein-like Polypeptide - A polypeptide displaying a human somatomedin regulating biological activity of the carrier protein and being capable of binding somatomedin-like polypeptides. Preferably, a carrier protein-like polypeptide displays a somatomedin-C regulating activity of the carrier protein. A carrier protein-like polypeptide may be a carrier protein subunit capable of binding somatomedin-like polypeptides, if it possesses such somatomedin regulating activity. This polypeptide may include one or more amino acids in addition to those of the carrier protein or such carrier protein subunits. This polypeptide may not include all of the amino acids of the carrier protein or such carrier protein subunits because one or more amino acids have been deleted or because one or more amino acids have been substituted for others. Thus, a carrier protein) like polypeptide may have the amino acid sequence of the carrier protein or of a carrier protein subunit in which an amino acid residue has been added, deleted or substituted. A carrier protein-like polypeptide may have the natural glycosyltion of the carrier protein, may lack the natural glycosylation of the carrier protein, or may have glycosyltion different from the natural glycosylation of the carrier protein. Thus, a carrier protein-like polypeptide may be unaccompanied by the associated natural glycosylation of the carrier protein. This polypeptide preferably has a molecular weight of about 40,000-50,000 daltons or less, if measured in a form accompanied by natural glycosylation. This polypeptide more preferably has a molecular weight of about 30,000 daltons or less, if measured in that form.

Somatomedin-C ("SM-C" or "IGF-I") - The principle hormone regulating growth after birth. SM-C mediates the growth promoting action of GH and binds to the carrier protein.

Nucleotide - A monomeric unit of DNA or RNA consisting of a sugar moiety (pentose), a phosphate, and a heterocyclic base. The four DNA bases are adenine ("A"), guanine ("G"), cytosine ("C"), and thymine ("T"). The four RNA bases are A, G, C, and uracil ("U").

DNA Molecule - A molecule other than the entire human genome composed of a sequence of nucleotides connected one to the other by phosphodiester bonds between the 3' and 5' carbons of adjacent pentoses. A DNA molecule may be composed of an isolated sequence of nucleotides that are part of the human genome. A DNA molecule may be composed of a single DNA molecule (commonly called "single stranded DNA") or two DNA molecules composed of complementary nucleotides (commonly called "double stranded DNA").

Recombinant DNA Molecule - A DNA molecule having at least one nucleotide sequence resulting from joining or adding together at least two DNA molecules.

Genome - The entire DNA of a cell or a virus. It includes the genes coding for the polypeptides of the organism, as well as operators, promoters and ribosome binding and other interaction sites.

Gene - A DNA molecule which encodes through its mRNA a sequence of amino acids of a specific polypeptide.

cDNA - A double-stranded DNA molecule produced from an RNA molecule by using that RNA as a template for RNA-directed synthesis of the first DNA strand followed by using that DNA strand as a template for DNA-directed synthesis of the second DNA strand.

Transcription - The process of producing mRNA from a gene.

Translation - The process of producing a polypeptide from mRNA.

Expression - The process of producing a polypeptide by transcription and translation.

Plasmid - A nonchromosomal double-stranded DNA molecule comprising an intact "replicon" such that the molecule is replicated in a host organism. When the plasmid is placed within a single celled organism, the characteristics of that organism may be changed as a result of the DNA of the plasmid. A cell transformed by a plasmid is called a "transformant."

Virus - DNA or RNA molecules in a protein envelope or coat capable of infecting a cell or organism.

Phage or Bacteriophage - Bacterial virus.

Vehicle or Vector - A plasmid, phage, mammalian virus, cosmid, or other DNA molecule which is able to be transformed into and to replicate in a host, having one or more sites at which such DNA molecules may be cut in a determinable fashion without loss of an essential biological function of the DNA, e.g., replication, production of coat proteins or loss of promoter or binding sites, and having a marker suitable for use in the identification of a transformed host, e.g., tetracycline resistance.

Cloning - A process of obtaining a population of organisms, cells or DNA molecules derived from one such organism, cell or DNA molecule.

Expression Control Sequence - A DNA sequence that controls and regulates expression of genes when operatively linked to those genes. They include the lac system, the trp system, the tac system, the trc system, major operator and promoter regions of phage λ, the T7 system, the control region of fd coat protein, the control sequences of SV-40, the actin system, the metallothionein system, the LTR (promoter-containing long terminal repeat of retroviruses) system, and other sequences known to control the expression of genes of prokaryotic or eukaryotic cells or organisms and their viruses or combinations thereof.

Host, Host Organism or Host Cell - A prokaryotic or eukaryotic cell or organism capable of being transformed by a vehicle or vector.

### Carrier Protein Subunits

The invention solves the problems referred to by making available human carrier protein subunits capable of binding somatomedin-like polypeptides. The ability of the carrier protein subunits of the invention to bind somatomedin-like polypeptides has been demonstrated by binding those subunits in vitro to somatomedin-C at about physiological pH. This binding activity demonstrates that the carrier protein subunits of the invention will bind somatomedin-like polypeptides in vivo, and provide substantially the transport and regulatory activity of the native carrier protein. When this description refers to the capability of the carrier protein subunits to bind somatomedin-like polypeptides, it is referring to this ability to bind such polypeptides in vitro or in vivo. The carrier protein subunits have no substantial binding activity for insulin.

The carrier protein subunits of the invention each constitute a single polypeptide chain. The carrier protein subunits of the invention have an N-terminal amino acid molecule of the formula: 
Gly-Ala-Ser-Ser-Ala-Gly-Leu-Gly-Pro-Val-
(1)                 (5)                 (10)
Val-Arg-R-Glu-Pro-R-Asp-Ala-Arg-Ala-
              (15)                 (20)
Leu-Ala-,
 wherein R is cysteine or half-cystine. Half-cystine refers to an amino acid bound to another half-cystine amino acid in the same polypeptide chain by a disulfide bond. Because the carrier protein may be polymorphic, the amino acid molecule of the carrier protein subunits may also vary depending on the polymorphic character of the carrier protein. For example, the carrier protein subunits may contain a glycine ("Gly") residue in place of the alanine ("Ala") at position 5 from the N-terminal. Similarly, the Glu at position 14 from the N-terminal may sometimes be replaced in part by Phe.

The carrier protein subunits of the invention have a range of molecular weights. The molecular weights of the carrier protein subunits referred to in this description are those determined by SDS-PAGE gel electrophoresis against proteins of known weight conducted in the presence of a suitable reducing agent such as β-mercaptoethanol "BME." The known protein standards were 200,000 (myosin (H-chain)), 97,400 (phosphorylase b), 66,200 (bovine serum albumin) 43,000 (ovalbumin), 25,700 (α chymotrypsinogen), 18,400 (β-lactoglobulin and 14,300 (lysozyme). Carrier protein subunits having molecular weights of about 15,000, 21,000, 26,000 and 30,000 daltons have been isolated and identified. The carrier protein subunits may differ in molecular weight because they were present in the carrier protein as polypeptides of that size or because of enzymatic digestion or break-down from other causes. Whatever the source of these differences, the carrier protein subunits of the invention have a molecular weight of about 30,000 or less. The carrier protein subunits of the invention preferably have a molecular weight of about 15,000 to and including about 30,000 daltons.

The carrier protein subunits are glycoproteins, as shown by their positive reaction to the periodic acid Schiff reagent and ability to bind concanavalin A cross-linked to agarose (Con-A Sepharose, Pharmacia). Binding to Con-A Sepharose is specific for glycoproteins containing glucose and mannose residues. Specific residues include α-D-mannopyranosyl and α-D-glucopyranosyl residues. Therefore, the carrier protein subunits are substantially glycosylated.

The invention also provides essentially pure carrier protein subunits having SM binding activity. The carrier protein subunits of the invention are essentially free of other proteins, peptides, nucleotides, polysaccharides, lipids and salts. By virtue of the invention, it is possible to obtain those subunits in sufficient purity for use in human and animal therapeutic agents, as animal growth promotion agents, in human and other animal diagnostic reagents, and in human and other animal research applications.

The invention also provides therapeutic compositions comprising an effective amount of at least one carrier protein subunit capable of binding somatomedin-like polypeptides, or pharmacologically acceptable salts thereof, and a pharmacologically acceptable carrier. The carrier protein subunit of such therapeutic compositions may be at least one essentially pure carrier protein subunit. Compositions of carrier protein subunits of the invention have many therapeutic uses involving the important biological properties of SMs. Compositions comprising the human carrier protein subunits may be useful in treatment of diseases involving increased, unregulated SM-dependent growth. Thus, the ability of the carrier protein subunits of the invention to inactivate SMs by binding permits a new therapy of several conditions. In such therapies, it is apparent that an effective amount of the carrier protein subunit is an amount sufficiently in excess of the biologically active, unregulated SMs to block or inactivate the SM activity. For example, an effective amount of carrier protein subunit may be 10 or more times the amount of biologically active SMs on a molar basis. For example, some cancers have been shown to produce SMs: fibro-sarcomas, chondrosarcomas and hepatoma cell lines. De Larco, J. E., et al., "A Human Fibrosarcoma Cell Line Producing Multiplication Stimulating Activity "MSA"-related Peptides," Nature, 272, pp. 356-358 (1978). Breast and renal cancers produce a SM which autostimulates the growth of the cancer. Spencer, E. M. et al., "Possible Auto-stimulation Of Human Mammary Carcinoma Growth By Somatomedins," Annals New York Academy Sciences, 464, pp. 448-449 (1986). Since endothelial cell and fibroblast proliferation are also stimulated by SMs, SMs produced by breast cancers can act also as a paracrine and stimulate the growth of the supporting stromal tissue critical to tumor survival. Bar, R. S., et al., "Receptors For Multiplication-stimulating Activity on Human Arterial and Venous Endothelial Cells, J. Clin. Endocrinol. Metab. 52, p. 814, (1981); Clemmons, D. R., et al., "Hormonal Control Of Immunoreactive Somatomedin Production By Cultured Human Fibroblasts." J. Clin, Invest 67, p. 10 (1981). By blocking the action of SMs, administering the human carrier protein subunit of the invention would be expected to reduce the rate of tumor growth and additionally render the malignant cells more sensitive to other drugs.

This invention also provides a composition comprising at least one carrier protein subunit substantially complexed with at least one somatomedin-like polypeptide. Such a composition would have a variety of therapeutic applications. SMs possess biological activity which make them potentially useful in many therapeutic applications. However, to maintain the required steady level of SMs in plasma, multiple daily injections would have to be given because the half-life of SMs may be less than one hour in the free condition. This obstacle cannot be overcome by administering a larger dosage because (a) SMs are potent mitogens for subcutaneous, muscular, and vascular tissues (fibroblasts, endothelial cells, muscle cells, adipocytes, and endothelial cells) and could produce local tissue proliferation, (b) large amounts of free SMs would cause hypoglycemia, and (c) the excessive amount of SMs required to maintain a steady plasma level would not be cost effective.

SM could be delivered to target tissues in a safe, effective physiologic manner and their half-life significantly prolonged by complexing them to the carrier protein subunits of the invention. The SM in a SM-carrier protein subunit complex would not be mitogenic at injection sites or hypoglycemic. This complex could be formulated to provide controlled, long-term absorption. After transport to target tissues, dissociation would release SM. Thus, therapy would mimic the physiologic delivery system. Successful therapeutic and animal husbandry use of SM-C, IGF-II and other somatomedin-like polypeptides are permitted by a composition of at least one human somatomedin-like polypeptide and at least one carrier protein subunit. Compositions comprising one or more carrier protein subunit and one or more SMs would also be useful for treatment of diseases such as postmenopausal osteoporosis, other forms of osteoporosis, and human GH deficiency, as well as for healing wounds and increasing animal growth. Such composition would be used to deliver SM to bony tissues and stimulate the growth of bone. Dissociation of the SM from the carrier protein subunit-SM complex should stimulate osteoblasts to increase bone formation in postmenopausal osteoporosis, invade the porous matrix of a prosthetic joint thereby stabilizing the prosthesis, and to promote healing of un-united fractures.

Therapeutic compositions comprising an effective amount of at least one carrier protein subunit capable of binding somatomodin-like polypeptides, or pharmacologically acceptable salts thereof, and a pharmacologically acceptable carrier and therapeutic processes using such compositions may also be useful in treating injuries or diseases in which the natural healing mechanism or response involves the presence of regulated levels of biologically active somatomedins. For example, such compositions may be useful in wound healing, where the natural physiological response involves the presence of endogenous SMs at the site of the wound. An effective amount of carrier protein subunit is an amount sufficient to prolong the half-life of the endogenous biologically active somatomedins.

For all of these reasons, there have been many attempts to determine the protein structure needed for carrier protein-like activity. None have identified and isolated the carrier protein subunits of this invention or isolated them in pure form.

### Recombinant DNA And Carrier Protein-Like Polypeptides

There will now be described methods for locating identifying, and isolating DNA molecules that code for carrier protein-like polypeptides, recombinant DNA molecules, vectors, hosts and methods for the use of those molecules, vectors and hosts in the production of carrier protein-like polypeptides, that is, polypeptides displaying a somatomedin regulating activity of a carrier protein and being capable of binding somatomedin-like polypeptides. Using these processes, it is possible to obtain carrier protein-like polypeptides for use in therapeutic and diagnostic compositions and methods. The production of these polypeptides is now possible in amounts and by methods not available previously. This invention also involves producing these polypeptides essentially, and more preferably completely, free of other polypeptides naturally present in human plasma.

As will be appreciated from the disclosure, the DNA molecules and recombinant DNA molecules of the invention contain genes that are capable of directing the expression, in an appropriate host. of carrier protein-like polypeptides. Replication of these DNA molecules and recombinant DNA molecules in appropriate hosts also permits the production in large quantities of genes coding for these polypeptides. The molecular structure and properties of these polypeptides and genes may thus be readily determined. The polypeptides and molecules are useful, either as produced in the host or after appropriate modification, in compositions and methods for improving the production of these products themselves and for use in therapeutic and diagnostic compositions and methods.

There is provided a DNA molecule comprising a gene which codes for a carrier protein-like polypeptide, namely one displaying a somatomedin regulating activity of the carrier protein and being capable of binding somatomedin-like polypeptides. Such a DNA molecule has been isolated in the sense that it is not the entire human genome. Such a DNA molecule is preferably free of introns. Such a DNA molecule is also preferably essentially free of genes which code for any other polypeptide coded for by the human genome. Preferably, such a gene codes for a polypeptide having a molecular weight of about 40,000-50,000 daltons or less, if molecular weight is measured in a form accompanied by natural glycosylation. Such a gene may code for a polypeptide displaying a somatomedin regulating activity of the carrier protein, and more preferably, a somatomedin-C regulating activity of the carrier protein. Such a gene may also code for a carrier protein-like polypeptide that is a carrier protein subunit capable of binding somatomedin-like polypeptides, and more preferably a carrier protein subunit capable of binding somatomedin-C.

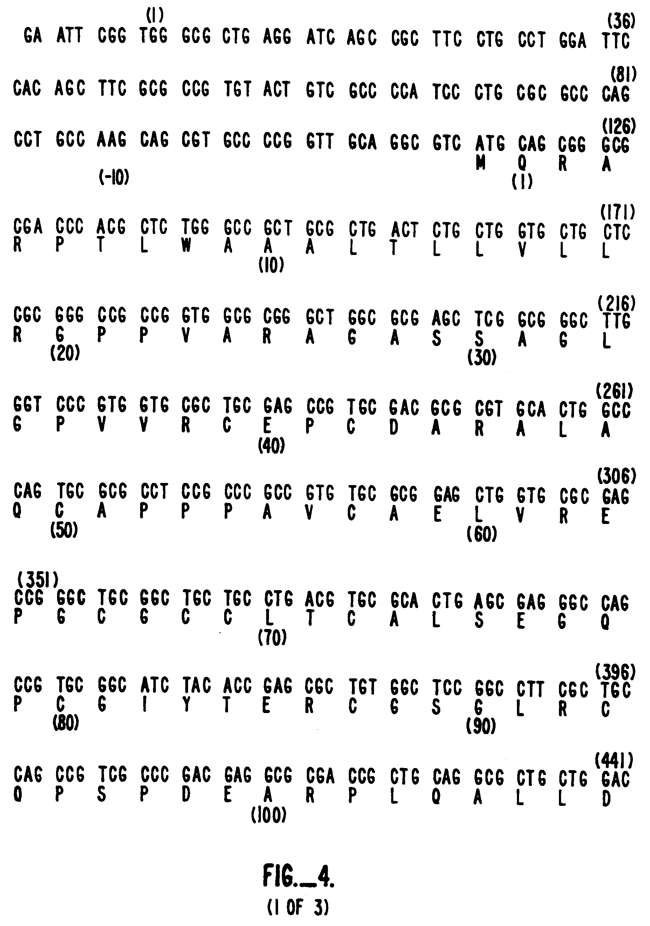

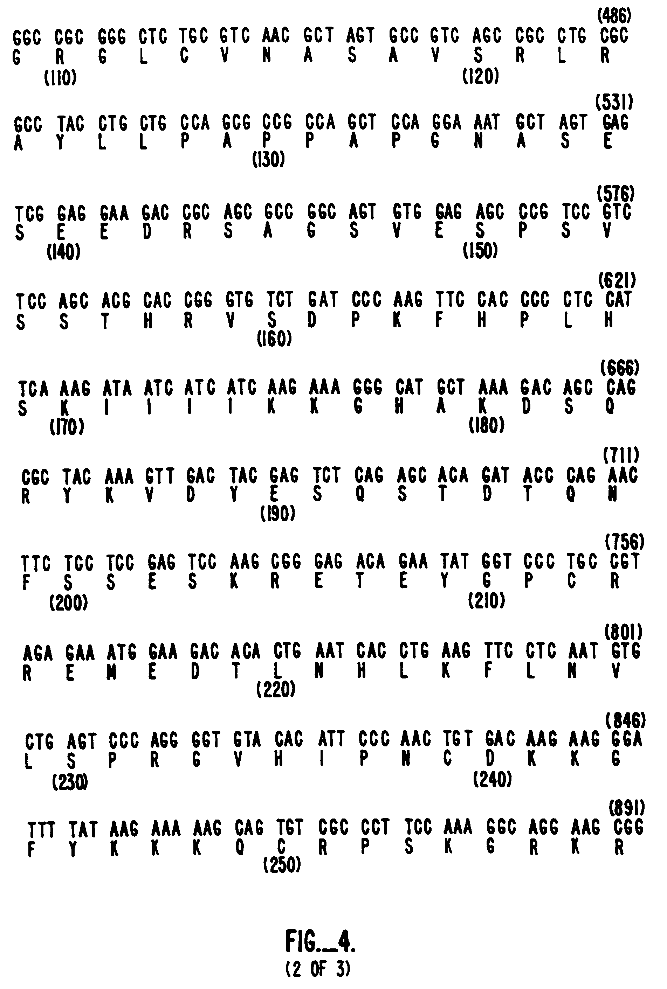

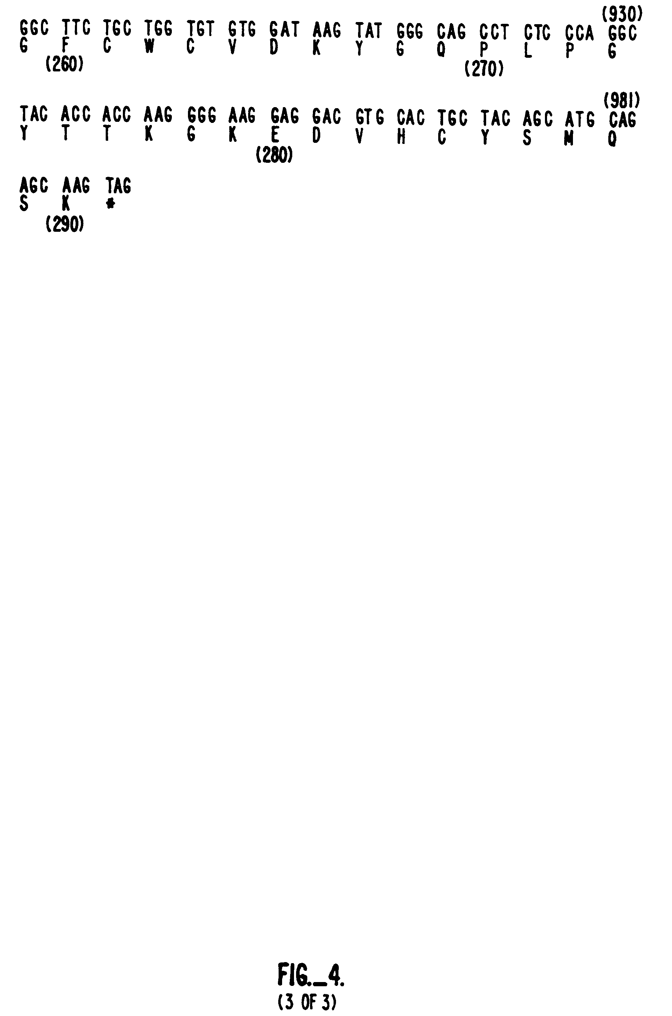

A process for obtaining such a DNA molecule, comprises preparing cDNA molecules from mRNA found in cells or tissues that produce the carrier protein, determining which of the cDNA molecules hybridize to one or more labelled polynucleotide probes based on the DNA sequence of Figure 4, analyzing the cDNA molecules that hybridized, and obtaining a DNA molecule having a gene which codes for a carrier protein-like polypeptide. In that process, a DNA molecule having the gene may be obtained by ligating one or more cDNA molecules that hybridized with other cDNA molecules, synthetic DNA molecules, or recombinant DNA molecules. The cDNA molecule which hybridizes to said probe may be a cDNA molecule selected from the group consisting of a human liver gene library, a human fibroblast gene library, a human placenta library, and a human epithelial library. In that process, the labelled polynucleotide probe may have the DNA sequence shown in Figure 2a. The invention also includes a DNA molecule made by that process, and a DNA molecule which encodes a carrier protein-like polypeptide coded for by a DNA molecule obtainable by that process.

An oligonucleotide probe having all or a portion of the DNA sequence of any one of the DNA molecules LCP, LCP 0.70, LCP 0.77, LCP 2.3, LCP 2.5, FCP 1.8 and FCP 2.5, which selectively hybridizes to a DNA molecule encoding a carrier protein-like polypeptide.

In addition, a DNA molecule of the invention may be selected from the group consisting of the DNA molecule LCP 0.70, LCP 0.77, LCP 2.3, LCP 2.5, FCP 1.8 and FCP 2.5, DNA molecules which hybridize to any of the DNA molecules LCP 0.70, LCP 0.77, LCP 2.3, LCP 2.5, FCP 1.8 and FCP 2.5, and which code for a carrier protein-like polypeptide, and DNA molecules which code for a polypeptide coded for by any of the foregoing DNA molecules. A preferred DNA molecule comprises a DNA molecule which is the carrier protein-related portion of LCP 2.3. Another recombinant DNA molecule comprises a DNA molecule which is the carrier protein-related portion of LCP 2.3, and DNA molecules which code for a polypeptide coded for by said portions of LCP 2.3.

Furthermore, a DNA molecule of the invention may comprise a gene which codes for a polypeptide having the sequence of amino acids -1 to 290 of Figure 4, amino acids 1 to 290 of Figure 4, or amino acids 27 to 290 of Figure 4. A DNA molecule may also comprise a gene which codes for a polypeptide having the sequence of amino acids 27 to 290 of Figure 4 and having a methionine residue preceding amino acid 27.

A DNA molecule may also comprise a gene which codes for a polypeptide having the sequence of amino acids 27 to 290 and having a sequence of amino acid residues preceding amino acid 27 that constitute a secretion, signal or other precursor sequence recognized by a host.

These DNA molecules may be used to construct a recombinant DNA molecule in which such DNA molecules are operatively linked to an expression control sequence. Preferably, such a recombinant DNA molecule constitutes a vector or vehicle. Methods for producing a vector comprising introducing into a vector a DNA molecule are known in the art. That method may comprise the additional step of introducing into said vector an expression control sequence, so as to control and to regulate the expression of that DNA molecule. The expression control sequence may be a lac system, a trp system, a tac system, a trc system, a T7 system, major operator and promoter regions of phage λ, the control region of fd coat protein, the control sequences of SV-40, the actin system, the metallothionein system, the LTR (promoter containing long terminal repeat of retrovirus) system, and other sequences which control the expression of genes or prokaryotic or eukaryotic cells and their viruses and combinations thereof.

The recombinant DNA molecules and vectors permit the production of carrier protein-like polypeptides in hosts. Also available are a host transformed with at least one of those recombinant DNA molecules or vectors. A transformed host may be strains of E. coli, Pseudomonas, Bacillus subtilis, Bacillus stearothermophilus, other bacteria, yeast, fungi, animal, insect or plant hosts and human tissue cells.

The carrier protein-like polypeptide of the invention may be prepared by a method comprising the steps of transforming an appropriate host with such a recombinant DNA molecule or vector, and culturing said host to make such a polypeptide. Preferably, the method includes the additional step of collecting said polypeptide. In this method, the host may be strains of E. coli, Pseudomonas, Bacillus subtilis, Bacillus stearothermophilus, other bacteria, yeasts, fungi, animal, insect or plant hosts, and human tissue cells. The method for producing such a polypeptide may also comprise the steps of culturing a host transformed by such a recombinant DNA molecule or vector.

The applicants have found a polypeptide that is coded for on expression by a recombinant DNA molecule or vector described above.

This provides an essentially pure carrier protein-like polypeptide other than a carrier protein subunit capable of binding somatomedin-like polypeptides. Such an essentially pure polypeptide is preferably essentially free of substances naturally present in human serum. Such a polypeptide may be a mature carrier protein-like polypeptide. Such a mature polypeptide is one in which the amino acid residues constituting a secretion, signal or other precursor sequence are deleted.

The present invention provides a carrier protein-like polypeptide which comprises a sequence having an in-vivo effector activity of a polypeptide with the sequence of Fig. 4 and lacking the natural glycosylation of the carrier protein. An example of such a polypeptide is the polypeptide with the sequence of Figure 4. Suitably the polypeptide is essentially free of substances naturally present in human serum.

The invention further provides an essentially pure polypeptide having the sequence of amino acids 27 to 290 of Figure 4 and having a methinonine residue preceding amino acid 27. One or more amino acid residues may be added, deleted or substituted provided the polypeptide constitutes a carrier protein-like polypeptide.

The invention further provides a polypeptide selected from the group consisting of a polypeptide having the sequence of amino acids -1 to 290 of Figure 4, and polypeptides having a portion of that sequence and constituting a carrier protein-like polypeptide.

Polypeptides of the invention may be substantially complexed with at least one somatomedin-like polypeptide.

The invention is also a therapeutic composition for inhibiting the effect of somatomedin-C for inhibiting the growth of keloid scars. The invention includes the use of the above described polypeptide for inhibiting the growth of somatomedin-dependent cancers in and for inhibiting the growth of keloid scars.

The invention is also embodied in a composition having at least one such carrier protein-like polypeptide described above substantially complexed with at least one somatomedin-like polypeptide. Such compositions may be used in a therapeutic composition for treating osteoporosis in humans. Such compositions may also be used in a method for treating such conditions comprising administering an effective amount of such a composition.

A recombinant DNA molecule may be provided having a DNA molecule including a gene which codes for such a carrier protein-like polypeptide linked to an expression control sequence and having a DNA molecule including a gene which codes for a somatomedin-like polypeptide operatively linked to an expression control sequence. A host may be transformed with at least one such recombinant DNA molecule to permit it to produce both types of polypeptides.

A single vector may also be constructed to contain a DNA molecule which codes for at least one carrier protein-like polypeptide described above and a DNA molecule which codes for a somatomedin-like polypeptide each operatively linked to an expression control sequence. A host may be transformed with such a vector. A method for producing a composition comprising a complex of a carrier protein-like polypeptide and a somatomedin-like polypeptide involves transforming an appropriate host with such a vector and culturing said host to make said polypeptides. That method could include the additional step of collecting the polypeptides. That method could comprise simply culturing a host transformed with such a vector. A method for producing such a composition also involves transforming an appropriate host with at least one recombinant DNA molecule or vector having a DNA molecule which codes for a carrier protein-like polypeptide as described above, co-transforming such host with at least one recombinant DNA molecule or vector having a DNA molecule which codes for a somatomedin-like polypeptide, and culturing such host to produce both types of polypeptides. The invention also encompasses hosts transformed with at least one of each such type of recombinant DNA molecule or vector.

### BRIEF DESCRIPTION OF THE DRAWINGS

Figure 1 shows the sequence of the N-terminal 42 amino acid residues of a human carrier protein subunit having a molecular weight of about 15,000 daltons. In addition, the N-terminal sequences of tryptic fragments T1, T6, T7, T1', and T10 are shown.

Figure 2a shows the sequence of the N-terminal 57 amino acids of the subunit referred to in Figure 1, and an oligonucleotide coding for amino acids 29-44, designed for use as a probe. This oligonucleotide is referred to as the 48mer.

Figure 2b shows the protein and DNA sequence of the 181-bp synthetic DNA and its corresponding protein sequence.

Figure 3a shows the size and restriction sites of the DNA inserts LCP 0.70 and LCP 0.77 that hybridized to the probe of Figure 2a, and the size and restriction sites of a DNA insert LCP 2.3 that hybridized to probes having the DNA sequences of those DNA inserts LCP 0.70 and LCP 0.77.

Figure 3b displays the strategy for sequencing LCP 2.3.

Figure 4 displays the nucleotide sequence of the coding strand of DNA molecule LCP 2.3 and the amino acid sequence of the 291 amino acids of the polypeptide for which it codes. "C" indicates a cysteine or half-cystine residue.

Figure 5 displays the functional and partial restriction map of vector pDJ4219 which contains a gene for a human carrier protein-like polypeptide having 264 amino acids inserted into pKK233-2, for expression in E.coli cells.

Figure 6 displays the preparation of various recombinant DNA molecules that may be employed in vectors for transforming appropriate hosts, which when cultured produce carrier protein-like polypeptides.

Figure 7 displays the functional and partial restriction map of vector pDJ4212 which contains a gene for a human carrier protein-like polypeptide having the first 120 amino acids of mature carrier protein that has been inserted into pSVL derivative pDJ4210, for expression in COS cells.

Figure 8 displays the functional and partial restriction map of vector pKG4403 which contains a gene for a human carrier protein-like polypeptide having 264 amino acids inserted into pKG3226, for expression in CHO cells.

Figure 9 shows the effect of l5kDa carrier protein subunit on the half-life of SM-C in circulation.

### Assay for Somatomedin Binding Activity

The somatomedin binding activity is measured by a protein binding assay employing a radiolabeled ¹²⁵I-SM (SM-C or IGF-II) as the ligand. The amount of ¹²⁵I-SM bound is compared to that of a standard preparation.

The standard was prepared by gel filtration of a pool of human serum from 10 normal donors. The serum, 35 ml, was added to 35 ml of 4 N acetic acid. After clarification, the sample was chromatographed on Sephadex G-50 (5 x 100 cm) (fractionation range 1,500 to 30,000) equilibrated with 1 M acetic acid at a flow rate of 80 ml/hr. All fractions were assayed for somatomedin binding activity using ¹²⁵I-SM-C. The binding activity appeared from K_{d} 0 - 0.4. These fractions were lyophilized, redissolved in 1 M acetic acid and rechromatographed to remove all traces of bound SMs. The final powder was redissolved in 35 ml of 0.1 M phosphate buffer pH 7.0, aliquoted in 100 ul amounts, and stored at -26°C. For each binding assay, a tube of this material was used as a reference that has arbitrarily been assigned a value of 1.0 U/ml.

The assay method was that described by Zapf et al., ("Serum Levels of the Insulin-like Growth Factor (SM) and its Carrier," Acta Endocrinol. 95, p. 505-517, (1980)). For samples where the carrier protein subunit was still complexed to SMs, the two were separated by Sephadex G-50 chromatography (0.9 x 110 cm) in 1 M acetic acid. The binding activity peak (K_{d}.1 - 0.4) was then lyophilized, reconstituted in assay buffer and tested. For samples that did not contain bound SMs, the samples were either dialyzed against assay buffer and tested directly or, if the concentration of binding activity was low, dialyzed vs 0.1 M acetic acid, lyophilized and dissolved in a smaller volume of assay buffer. The assay buffer was 0.1 M sodium phosphate pH 7.0 containing 0.2% human or bovine serum albumin which had been previously tested to ensure absence of competing activity. SM-C or IGF-II were iodinated by the method of Spencer. Grecu, E. O., E. M. Spencer, et al., "Serum Somatomedin Response to Human Growth Hormone Infusion in Patients with Diabetes Mellitus; Correlation with the Degree of Control of Diabetes," Am. J. Med. Sci., 287, pp. 7-10 (1984). Serial dilutions (2-or 4-fold) of samples and standard were assayed in triplicate. Assay tubes consisted of 100 ul of ¹²⁵I-SM, 20,000 cpm, and 200 ul of the sample. The assay was carried out at 4°C for 16 h although satisfactory results could be obtained with a 2 h incubation at room temperature. The bound ¹²⁵I-SM was separated from the free by charcoal extraction. An ice cold solution, 0.8 ml, of 2% activated charcoal with 1% human (or bovine) albumin in 0.1 M phosphate buffer pH 7.0 was added and the tubes vortexed for 15 minutes at 4°C. After centrifugation, the supernatant was counted. The cpm bound were plotted against the log of the dose and the potency of the unknown related to that of the standard assigned a value of 1.0 U/ml. The specificity of binding was determined by incubating the sample with a large excess of an unlabeled SM.

### Other Somatomedin Binding Assays

Dot-blot and Western assays may also be used to determine the existence of polypeptides with somatomedin binding activity.

### Dot Blot "Binding In Wells" Format

The nitrocellulose membrane and 3-MM filter paper are first placed in water and subsequently soaked in PBS (10 mM NaPO₄, pH 7.2, 0.15 M NaCl) for 20-30 minutes. The filter paper and membrane are placed on the dot-blot apparatus, with the membrane on top of the filter paper. The apparatus is assembled and clamped according to manufacturer's instructions (Bio-Rad). The dot-blot apparatus contains 96 wells which makes it very convenient to process many samples simultaneously. Wells are rinsed with 200 ul PBS. Carrier protein-like polypeptides are diluted in PBS to the appropriate concentrations to make total volumes of 50 ul/well. Control and blank wells contain BSA (bovine serum albumin) or no protein, respectively. Samples are applied to wells and are allowed to flow through the membrane by gravity. Binding of the protein to the membrane is completed within 30-60 minutes. The membrane is blocked with 200 ul/well 1% BSA in PBS, which is allowed to flow by gravity for 30 minutes, then is "pulled" by a vacuum through the membrane. Wells are washed three times with 100 ul TBS (50 mM Tris-HCl, pH 7.5, 0.15 M Nacl), 0.1% Tween 20. ¹²⁵I-SM-C (20,000-200,000 cpm) is added in 50 ul PBS per well. The apparatus is tightly covered with Parafilm and left at 4 for 1.5-2 hours. This step constitutes the binding of SM-C to carrier protein-like polypeptides. The apparatus is disassembled and the membrane washed in large volumes of TBS; TBS, 0.1% Tween 20; and TBS; each wash is 15 minutes at 4°C with gentle shaking. The membrane is air dried and exposed to Kodak X-Omat AR film with intensifying screens at -70°C for 1-6 hours.

### Dot Blot "Binding in Bag" Format

Pretreatment of membrane, dot-blot apparatus assembly, and binding of protein to membrane is carried out as described above. Following binding of protein to membrane, the dot-blot apparatus is disassembled, and the membrane is air dried. The membrane is placed in a dish and washed at 4 with gentle shaking in the following solutions: TBS plus 3% NP40, for 30 minutes; TBS plus 1% BSA, for 1 hour; TBS plus 0.1% Tween 20, for 10 minutes. The membrane is placed in a bag with 6-10 ml binding solution (TBS, 1% BSA, 0.1% Tween 20). ¹²⁵I-SM-C (2-20 million cpm) is added and binding proceeds at 4°C for 2 hours or overnight, with gentle shaking. This step constitutes the binding of SM-C to carrier protein-like polypeptides. The membrane is washed two times in large volumes of TBS, 0.1% Tween 20 and two times in TBS alone. Each wash is done for 15 minutes at 4°C, with gentle shaking. The membrane is air dried and exposed to Kodak X-Omat AR film with intensifying screens at -70°C for 5-16 hours.

### Western

Protein samples containing carrier protein-like polypeptides are loaded and run on polyacrylamide-SDS gels. Normally 12% gels are run which will allow for good separation of proteins between 10,000 and 70,000 daltons. Separation is accomplished by electrophoresis. Proteins within the gel are then blotted onto a nitro-cellulose membrane, and the resultant membrane is air dried 5 minutes at 37°C. The membrane, containing the bound proteins, is rinsed with TBS plus 3% NP40 at 4°C for 30 minutes. The membrane's nonspecific sites are blocked with 1% BSA in TBS at 4°C for 2 hours. The membrane is rinsed with TBS plus 0.1% Tween 20 at 4°C for 10 minutes. The membrane is probed with ¹²⁵I-SM-C by placing the membrane in a bag with 6-10 ml TBS, 1% BSA, 0.1% Tween 20 plus 500,000 cpm ¹²⁵I-SM-C. The membrane is gently shaken overnight at 4°C to allow for binding between SM-C and carrier protein-like polypeptides immobilized on the membrane. The membrane is subjected to the following washes at 4°C: TBS plus 0.1% Tween 20, twice, for 15 minutes each; TBS, three times, for 15 minutes each. The membrane is air dried and exposed to Kodak X-Omat AR film with intensifying screens at -70°C for 5-16 hours.

### Process For Producing Carrier Protein Subunits From Plasma

The procedure for producing the carrier protein subunits began with Cohn fraction IV-1. This is a human plasma fraction that contains about 10% of the plasma proteins and 40% of the original plasma carrier protein activity. It is a green-yellow paste, approximately 35% solids, much of which are denatured insoluble proteins and glycoproteins. Each kilogram of this paste contains approximately 10 mg of carrier protein.

All assay buffers described below contained the following enzyme inhibitors, unless otherwise noted: 1 millimolar ("mM") phenylmethylsulfonyl fluoride ("PMSF"), 1 mM N-ethylmaleimide ("NEM"), and 1 mM ethylenediaminetetraacetic acid ("EDTA"). Enzyme inhibitors were essential because either the carrier protein has inherent protease activity or at least one other plasma protease was co-purified through the affinity chromatography step.

### Preparation 1

The carrier protein subunit was purified using gel filtration. A 30 mg aliquot of the resulting sample containing SM binding activity was dissolved in 0.1% TFA solution and injected into a preparative Vydak C4 RP column. The column was eluted with a 0-60% acetonitrile gradient in 60 minutes. The SM binding activity peak which eluted at approximately 39% acetonitrile was collected and lyophilized. The SM binding activity was recovered quantitatively.

The sample was subsequently resuspended in a Tris-glycine buffer containing β-mercaptoethanol and separated by SDS-PAGE (12.5% polyacrylamide). Bands corresponding to 15, 21, 26, and 30 kDa carrier protein subunits (each of which bound labelled SM in a Western blot) were cut from the gel, and the proteins were electroeluted into Tris-glycine buffer. Each of the carrier protein subunits was lyophilized; recoveries were quantitative.

### Example 1

Experiments designed to measure the potential of SM carrier protein subunits to potentiate wound healing were carried out in the following manner. Each of 6 anesthetized 300 gram male Sprague-Dawley rats was implanted subcutaneously (s.c.) with Schilling-Hunt wire mesh wound cylinders in each of the 4 quadrants on their back. Cylindrical chambers, 20 X 5.8 mm i.d. with a volume of 520 ul, were constructed out of stainless steel wire mesh. One end was sealed with wire mesh and the other with a silastic disk. After implantation, the typical progression of wound healing events occurred: thrombosis of blood vessels followed sequentially by migration through the wire mesh of polymorphonuclear leukocytes, macrophages and fibroblasts, with subsequent fibroplasia, collagen synthesis and angio-genesis. During this process, the wound fluid that collected in the hollow chamber could be sampled or injected with active agents (s.c. through the silastic disk). Most of the healing was complete by 17 days after implantation; however, the central cavity was never completely obliterated.

The 15 kDa SM carrier protein subunit was dissolved in PBS (150 mM sodium chloride, 10 mM sodium phosphate, pH 7.4), containing 0.1% bovine albumin. The wound chambers were injected with 100 ul of this solution (containing 1.4 ug of the 15 kDa species) every 12 hours. This amount was selected to be only slightly in excess of the amount of biologically active somatomedins and thereby increase the half-life of somatomedins present. After 17 days, wound cylinders were removed, and the fibrous tissue was scraped carefully from each cylinder. Cylinders injected with 15 kDa carrier protein subunit material were all filled with dense fibrous tissue that was considerably greater than that in the controls. Specifically, 19.5 ± 7 (SD) mg of protein were deposited in wound chambers containing 15 kDa carrier protein subunit as compared to 7.0 ± 1.6 mg deposited in controls. DNA synthesis was also much greater in carrier protein subunit-containing chambers (1160 ± 200 ug vs 380 ± 15 ug in controls). Likewise, hydroxyproline levels (an indicator of collagen synthesis) were significantly higher in carrier protein subunit-containing chambers (460 ug vs 270 ug in controls).

These results demonstrate that injection of 15 kDa carrier protein subunit into wound chambers markedly augments the rate of healing.

### Example 2

An animal experiment was conducted to show that the carrier protein subunits increase the serum half-life of SM-C. The 15 kDa human carrier protein subunit was shown to prolong the half-life of purified human SM-C injected into a rat's bloodstream.

The complex between the 15 kDa carrier protein subunit and ¹²⁵I-SM-C was formed by incubating ¹²⁵I-SM-C with the carrier protein subunit overnight at 4°C in PBS (10 mM sodium phosphate, pH 7.25, 150 mM sodium chloride). The complex was separated from free ¹²⁵I-SM-C by gel filtration. Specific activity of the ¹²⁵I-SM-C was 6.7 x l0⁵ cpm per ug.

Rats (about 200 grams) were anesthetized and catheterized through the jugular vein. Prior to injections, the catheters and syringes were rinsed with 4% BSA (bovine serum albumin) to prevent sticking of the proteins to plastic surfaces. Four rats received BSA, four rats received 2 ug ¹²⁵I-SM-C alone, and four rats received 2 ug ¹²⁵I-SM-C complexed with 15 kDa carrier protein subunit. Both the complex and the SM-C were in PBS. One rat received 1 ug ¹²⁵I-SM-C complexed with the carrier protein subunit. Blood samples (100-200 ul) were removed at multiple time points post injection. Blood cells were immediately separated from the plasma by centrifugation. A 25 ul plasma aliquot was counted to determine the concentration of ¹²⁵I-SM-C present and a 10 ul aliquot was run on a 15% polyacrylamide-SDS gel to determine SM-C integrity. Injections were carried out over a two day period. Each morning 2 rats were injected with the complex and 2 rats with SM-C alone. On a third day, 4 control rats were injected with BSA.

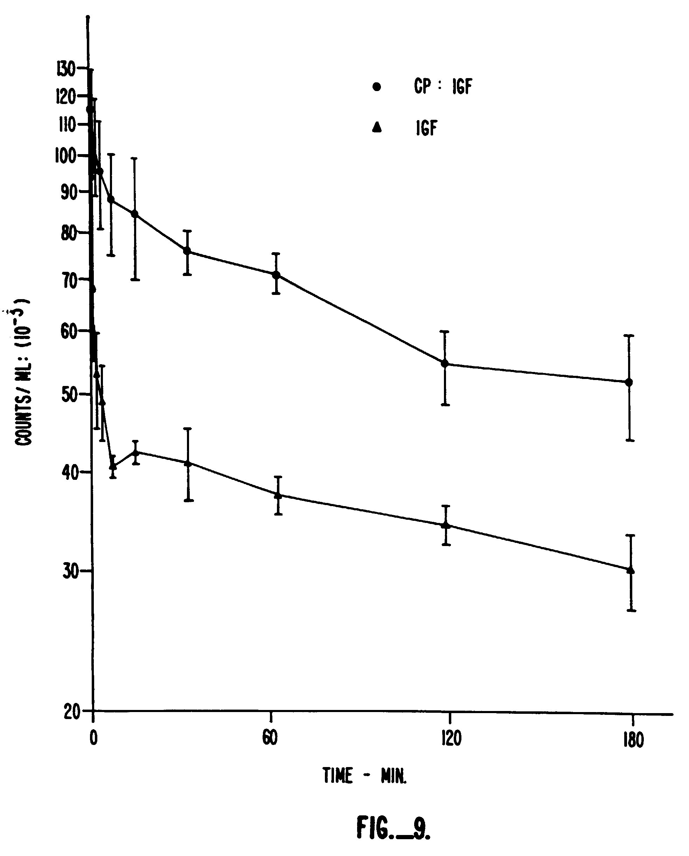

This study demonstrates that the 15 kDa carrier protein subunit significantly increases the half-life of SM-C in the circulation. An equal number of counts (i.e., 1.3 x 10⁵ cpm/ml rat blood) of SM-C was added to rats either alone or complexed with the carrier protein subunit. As shown in Figure 9, a majority of free SM-C is rapidly removed from the circulation, whereas the carrier protein subunit protects SM-C from that removal. (Samples run on 15% SDS [sodium dodecyl sulfate] polyacrylamide gels indicated that all ¹²⁵I counts were SM-C; that is, there is no free ¹²⁵I interfering with the experiment.) The continued appearance of the residual amount of free SM-C after 7.5 minutes may be due to SM-C occupying unsaturated rat carrier protein subunit molecules. Obviously, there were not sufficient endogenous carrier protein subunits to bind even 30% of all the free SM-C injected. It should be noted that there are not sufficient endogenous unsaturated carrier protein subunits in rats or in humans to be therapeutically useful. Thus, SM-C must be administered complexed to its carrier protein subunit.

### Recombinant DNA And Carrier Protein-like Polypeptides

### Preparation Of Oligonucleotide Probes Based On Protein Sequence Information

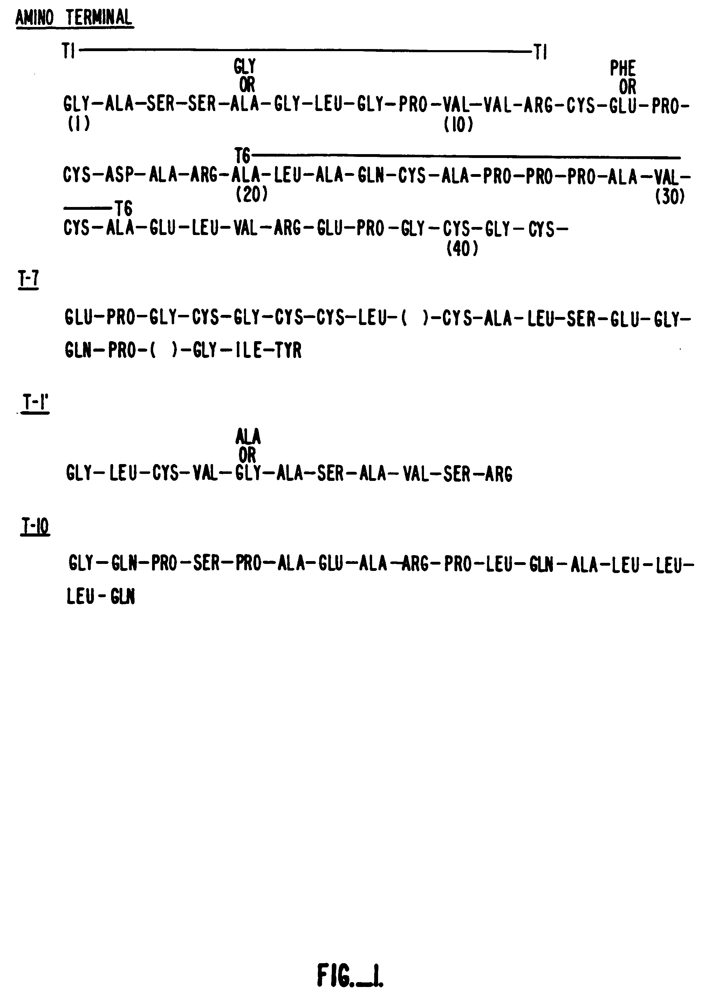

The carrier protein contains subunits that may be isolated and retain the capability of binding somatomedins, including subunits having apparent molecular weights, if glycosylated, of about 15, 21, 26, 30 and 45 kDa, and significantly less, if not glycosylated or subjected to other post translation modifications. If the N-terminal sequences of the subunits are the same, and the various subunits are encoded by the same gene or genes, then it should be possible to prepare a probe based on a common N-terminal sequence to identify DNA coding for carrier protein-like polypeptides. A carrier protein subunit was isolated and purified as described in Example 2, identified as S-15. The protein, S-15, was carboxymethylated and subjected to N-terminal sequence analysis using an Applied Biosystems Gas Phase Protein Sequencer, Model 470, by automated Edman degradation. The first 42 amino acids are in Figure 1. In addition, the subunit S-15 was cleaved with the protease trypsin which specifically cleaves after arginines and lysines, unless lysine is followed by proline. Specifically, carboxymethylated S-15 was digested with trypsin in 0.3 M sodium bicarbonate, pH 8.0. Tryptic fragments were separated by reverse phase HPLC using a Vydac C₄ column. Purified fragments were collected and sequenced as described above. The sequences of several such tryptic fragments, denoted as T-1, T-6, T-7, T-1', and T-10, are also shown in Figure 1. Due to the homology between the amino terminus and tryptic fragment T-7, it was determined that the first 57 N-terminal amino acids of subunit S-15, with two undetermined amino acids, are as shown in Figure 2a.

Many oligonucleotides were designed from this molecule to serve as probes to screen cDNA libraries. These included short degenerate probes and long codon biased probes. One oligonucleotide corresponding to a portion of the N-terminal 57 amino acid molecule identified as the 48mer, is shown in Figure 2a.

### Selection Of Tissues For Preparation Of PolyA⁺ RNA Containing Carrier Progein mRNA

The strategy utilized to isolate carrier protein genes was to identify a tissue making large quantities of carrier protein, isolate mRNA from that tissue, construct a cDNA library from that mRNA, and screen for the gene using oligonucleotide probes. The hope was that an enriched cDNA library would contain more copies of such a gene than would a genomic (total DNA) library which will only contain perhaps one copy. There was no information in the literature to establish which tissue or cell type makes the carrier protein, a protein which is found in the serum. Fibroblasts had been shown to produce small amounts of a large but otherwise uncharacterized somatomedin binding protein (Adams, et al, supra). However, it is known that the majority of SM-C is synthesized in the liver. In addition, SM-C is synthesized by fibroblasts and other tissues such as the heart, bone, placenta, and kidney. Therefore, speculating that SM-C and the carrier protein would be synthesized by the same tissues, the liver and fibroblast cells were chosen as two potential sources of the mRNA coding for the carrier protein.

In order to identify a tissue or cell line source of such mRNA, RNAs isolated from several human livers were prepared and tested for their ability to direct the synthesis of carrier protein. In addition, various fibroblast cell lines were assayed for their ability to make carrier protein.

### Preparation Of PolyA⁺ Containing RNA

Total and polyA⁺ containing RNA were isolated from various liver tissues and fibroblast cells according to standard procedures (Chirgwin, J.M., Pryzbyla, A.E., MacDonald, R.J. & Rutter, W.J. (1979) Biochemistry 18, 5294-5299 and Iversen, P.L., Mata, J.E. & Hines, R.N. (1987) BioTechniques 5, 521-523.). Either tissue (e.g., liver) or cells (e.g., fibroblasts) were homogenized in GIT buffer (4 M guanidinium isothiocyanate, 20 mM EDTA, 100 mM Tris-HCl, pH 7.6). Debris was removed, and the RNA-containing supernatant was brought to 2% Sarkosyl (sodium laurel sarkosinate) and 1% β-mercaptoethanol. The mixture was then centrifuged through a cesium chloride gradient. Pellets were resuspended and extracted with phenol and chloroform and subsequently precipitated with ethanol. PolyA⁺ RNA, which represents the mRNA, was purified from total RNA by passing total RNA over an oligo-dT cellulose column (Aviv. H. & Leder, P. [1972] PNAS 69:1408). The resulting polyA⁺ containing RNA was eluted from the column with 10 mM Tris, pH 7.4, 1 mM EDTA, 0.05% sodium dodecyl sulfate (SDS), concentrated, and stored for further use. The liver polyA⁺ RNAs were assigned the names H10 and H14, indicative of the liver sample from which they were purified, and the fibroblast cell polyA⁺ RNAs assigned the code name W138, HS27, MRC5, 8387, and MDA-MB-231 indicative of the cell source of the RNA.

### Testing Of RNA For Translation Products

An aliquot of human liver polyA⁺ RNA from H10 and H14 were translated in vitro using a rabbit reticulocyte translation kit with ³⁵S-methionine according to standard procedures (Davis, L. G., et al., "Basic Methods in Molecular Biology," (Elsevier, New York, NY, 1986)). The protein translation products were immunoprecipitated (according to Davis) with an antibody provided by Robert C. Baxter (Royal Prince Alfred Hospital, Australia), prepared in accord with Martin, J.L., et al. "Antibody Against Acid-Stable Insulin-like Growth Factor Binding Protein...", J. Clin. Endocrinol. Metab., 261, pp. 799-801 (1985). That antibody was raised against material containing the so-called acid-stable subunit of the carrier protein obtained from human serum. Immunoprecipitated proteins were analyzed by SDS-polyacrylamide gel electrophoresis. Protein bands of about 68,000, 43,000, 39,000 and 32,000 daltons were identified that reacted specifically with anti-carrier protein subunit antibody. The proteins were not precipitated by a control serum, which did not contain anti-carrier protein subunit antibodies. This result suggested that carrier protein is being made by a liver and that a cDNA library made from liver mRNA should contain the carrier protein gene.

Several fibroblast cell lines were also tested for their ability to produce the carrier protein. For example, WI38 embryonic fibroblasts (American Type Culture Collection No. CCL-75) were grown to 70-80% confluence in DMEM-F12 media containing 10% fetal calf serum. Cells were switched to serum free media and incubated for 72 hours. Culture supernatants were harvested and concentrated by TCA precipitation or by centrifugation. Samples were subjected to SM-Western analysis (SDS-PAGE step being carried out under non-reducing conditions) which demonstrated that WI38 cells synthesized and secreted at least 4 proteins capable of binding SM-C, in the size range of 25,000-45,000 daltons. Of these, an about 40,000 dalton protein (by reducing SDS-PAGE) was also specifically recognized by the anti-carrier protein subunit antibody. In this experiment, the 72 hour incubation of WI38 cells in serum free medium included the addition of ³⁵S-cysteine. The proteins were immunoprecipitated with anti-carrier protein subunit antibody and analyzed by SDS-PAGE under reducing conditions.

Other cell lines encoding carrier protein subunits that were both recognized by anti-carrier protein subunit antibody and bound by SM-C include HS27 (human fibroblast), MRC5 (human fibroblast), 8387 (human fibrosarcoma), and MDA-MB-231 (human breast cancer). It is expected that polyA⁺ RNA isolated from other fibroblast lines would also encode carrier protein.

It should be recognized that the polyA⁺ RNA product obtained from these sources contain a very large number of different mRNAs. Except for the mRNA specific for carrier protein or carrier protein subunits, the other mRNAs are undesirable contaminants. Unfortunately, these contaminant RNAs may behave similarly to carrier protein subunit mRNA throughout the remainder of the cloning process of this invention. Therefore, their presence in the polyA⁺ RNA will result in the ultimate preparation of a large number of unwanted bacterial clones, which contain genes that may code for polypeptides other than carrier protein. This contamination presents complex screening problems in the isolation of the desired carrier protein hybrid clones. In the case of carrier protein, the screening problem was further exacerbated by the lack of a sufficiently purified sample of carrier protein mRNA or DNA, or portion thereof, to act as a screening probe for the identification of the desired clones. The only available probes were those based on the limited N-terminal protein molecule information. Therefore, the screening process for the carrier protein clones is very time-consuming and difficult. Furthermore, because only a very small percentage of carrier protein clones themselves are expected to express carrier protein-like polypeptide in a biologically or immunologically active form, the isolation of an active clone is a difficult screening process.

### Synthesis Of Double Stranded cDNA Containing Carrier Protein cDNA

PolyA⁺ RNA containing carrier protein mRNA was used as a template to prepare complementary DNA ("cDNA"), essentially as described by Gubler and Hoffman. cDNA libraries were made from the mRNAs which had been shown to encode potential carrier protein-like polypeptides. The libraries were constructed in the λ vector gt10, but could be constructed in other vectors as well (e.g., λ gt11 [Young, R.A. & Davis, R.W. (1983) Proc. Natl. Acad. Sci. USA 80, 1194-1198]). Double-stranded cDNA was generated essentially according to the Gubler-Hoffman method (Gubler, U. & Hoffman, B.J. (1983) Gene 25, 263-269). In this protocol, first strand cDNA was synthesized using Moloney Reverse Transcriptase to copy the polyA⁺ RNA. Libraries described below include a random-primed human liver cDNA library (H14), two oligo-dT-primed human liver cDNA libraries (H14, H10/H14 [a pool of H10 and H14]), and an oligo-dT-primed human embryonic fibroblast library (WI38). Random primers (pd(N)₆) and oligo-dT (pT₁₂₋₁₈) primers were obtained from Pharmacia. The second strand was produced using a combination of RNAseH and DNA polymerase I.

The resulting cDNA population is in fact a complex mixture of cDNAs originating from the different mRNAs, which were present in the polyA⁺ RNA. In addition, because of premature termination by Moloney reverse transcriptase, many of the cDNAs are incomplete copies of the various mRNAs in the polyA⁺ mRNA.

### Cloning Of Double-Stranded cDNA

A wide variety of host vehicle combinations may be employed in cloning or expressing the double-stranded cDNA prepared as described above. For example, useful cloning or expression vehicles may consist of segments of chromosomal, non-chromosomal and synthetic DNA molecules, such as various known derivatives of SV40 and known bacterial plasmids, e.g., plasmids from E. coli including col El, pCR1, pBR322, pMB9 and their derivatives, wider host range plasmids, e.g., RP4, phage DNAs, e.g., the numerous derivatives of phage λ, e.g., NM 989, and other DNA phages, e.g., M13 and Filamenteous single-stranded DNA phages and vectors derived from combinations of plasmids and phage DNAs such as plasmids which have been modified to employ phage DNA or other expression control molecules or yeast plasmids such as the 2 µ plasmid or derivatives thereof. Useful cloning or expression hosts may include bacterial hosts such as E. coli HB 101, E. coli X1776, E. coli X2282, E. coli MRCI, E. coli LE392, E. coli C600 and strains of Pseudomonas, Bacillus subtilis, Bacillus stearothermophilus and other bacteria, yeasts and other fungi, animal, insect or plant cells. Of course, not all host/vector combinations may be equally efficient. The particular selection of host vehicle combination may be made by those of skill in the art after due consideration of the principles set forth herein without departing from the scope of this invention.

Furthermore, within each specific cloning or expression vehicle, various sites may be selected for insertion of the double-stranded DNA. These sites are usually designated by the restriction endonuclease which cuts them. These sites are well recognized by those of skill in the art. It is, of course, to be understood that a cloning or expression vehicle useful in this invention need not have a restriction endonuclease site for insertion of the chosen DNA fragment. Instead, the vehicle could be joined to the fragment by alternative means.

The cloning or expression vehicle or vector, and in particular the site chosen therein for attachment of a selected DNA fragment to form a recombinant DNA molecule, is determined by a variety of factors, e.g., number of sites susceptible to a particular restriction enzyme, size of the protein to be expressed, susceptibility of the desired protein to proteolytic degradation by host cell enzymes, contamination or binding of the protein to be expressed by host cell proteins difficult to remove during purification, expression characteristics, such as the location of start and stop codons relative to the vector molecules, and other factors recognized by those of skill in the art. The choice of a vector and an insertion site for a particular gene is determined by a balance of these factors, not all selections being equally effective for a given case.

Although several methods are known in the art for inserting foreign DNA into a cloning vehicle or expression vector to form a recombinant DNA molecule, the method preferred for initial cloning in accordance with this invention is digesting λ gt10 with EcoRI. The double-stranded cDNA is then ligated to this λ gt10 DNA, after first adding EcoRI linkers to the cDNA molecules. The resulting recombinant DNA molecule now carries an inserted gene at the chosen position in the cloning vector.

Of course, other known methods of inserting DNA molecules into cloning or expression vehicles to form recombinant DNA molecules are equally useful in this invention. These include, for example, dA-dT tailing, direct ligation, synthetic linkers, exonuclease and polymerase-linked repair reactions followed by ligation, or extension of the DNA strand with DNA polymerase and an appropriate single-stranded template followed by ligation.

It should, of course, be understood that the nucleotide molecules of cDNA fragments inserted at the selected site of the cloning vehicle may include nucleotides which are not part of the actual gene coding for the desired polypeptide or may include only a fragment of the complete gene for the desired protein. It is only required that whatever DNA molecule is finally inserted, a transformed host will produce a polypeptide having a somatomedin regulating biological activity of the carrier protein and being capable of binding somatomedin-like polypeptides, or that the DNA molecule itself is of use as a hybridization probe to select clones which contain DNA molecules useful in the production of polypeptides having such biological and binding activity.

The cloning vehicle or expression vector containing the foreign gene is employed to transform a host so as to permit that host to express carrier protein-like polypeptides. The selection of an appropriate host is also controlled by a number of factors recognized by the art. These include, for example, compatibility with the chosen vector, toxicity of proteins encoded by the hybrid plasmid, ease of recovery of the desired protein, expression characteristics, safety and cost. A balance of these factors must be struck with the understanding that not all hosts may be equally effective for either the cloning or expression of a particular recombinant DNA molecule.

In the present synthesis, the preferred initial cloning vehicle is λ gt10 and the preferred initial restriction endonuclease site is EcoRI. The preferred initial host is E. coli.

EcoR1-restricted λ gt10 DNA (Promega) was ligated to EcoR1 linkered cDNA molecules prepared as described in Maniatis, T., et al., Molecular Cloning: A Laboratory Manual, (Cold Spring Harbor Laboratory, Cold Spring Harbor, NY, 1982) and Davis, L. G., et al., "Basic Methods in Molecular Biology," (Elsevier, New York, NY 1986) ("Maniatis").

The hybrid DNA obtained after annealing is, of course, a large mixture of different recombinant DNA molecules and some cloning vehicles without inserted DNA molecules. However, each recombinant DNA molecule contains a cDNA segment at the EcoRI site. Each such cDNA segment may comprise a gene or a fragment thereof. Only a very few of the cDNA fragments code for carrier protein or a portion thereof. The vast majority code for one of the other proteins or portions thereof whose mRNAs were part of the polyA⁺ RNA used in the process of this invention. It should also be understood that it is possible that none of the clones of the above-prepared library may permit the expression of carrier protein-like polypeptides. Instead, they may only be useful in screening for and identifying such a clone.

The resultant λ DNA vectors containing cDNA inserts were packaged into λ phage using a λ phage packaging kit (Stratagene).

E. coli cells (e.g., C600 hf1) were infected with the recombinant phage and plated onto enriched media plates, (e.g. LB). Plates were incubated at 37°C until phage plaques were visible.

The phage plaques (clones) contain a variety of recombinant DNA molecules representing sized, complete or partial copies of the mixture of polyA⁺ RNA obtained from the liver. Each of the majority of these plaques will contain a single recombinant DNA molecule. However, only a very few of these recombinant DNA molecules are related to carrier protein. Accordingly, the clones must be screened to select the carrier protein related clones from the others.

### Screening For A Clone Containing Carrier Protein cDNA

There are several approaches to screen for clones containing carrier protein cDNA. These include, for example, RNA selection hybridization, differential hybridization; hybridization with a synthetic probe or screening for clones that produce the desired protein by immunological or biological assays. We have chosen hybridization with a synthetic probe as being the most convenient and promising method for primary clone screening.

There is no assurance that the recombinant DNA molecules and bacterial cultures transformed therewith, which are identified by hybridization with a probe, contain the complete carrier protein cDNA molecule or that the DNA molecule actually codes for carrier protein or will permit the clone to express a carrier protein-like polypeptide. However, the recombinant DNA molecules will certainly contain extensive nucleotide molecules complementary to the carrier protein subunit mRNA coding molecule. Therefore, the recombinant DNA molecule may at least be used as a source of a probe to screen rapidly other recombinant DNA molecules and clones transformed with them to identify further sets of clones which will contain an authentic or complete carrier protein subunit nucleotide coding molecule. These clones may then be analyzed directly for possible expression of polypeptides displaying the biological and binding activity of carrier protein. More importantly, the nucleotide molecule of the inserted DNA fragment of these hybrid plasmids and its amino acid translation product may be determined using conventional means and that DNA molecule used to construct appropriate expression vectors that permit the synthesis of carrier protein-like polypeptides in appropriate hosts transformed with them.

### Oligonucleotide Probe Hybridization

The phage cDNA library was mixed with E. coli and plated onto LB (enriched media) plates. The plates were incubated at 37°C until phage plaques were visible. Each plaque represents a clone of a unique λ gt10 phage containing a cDNA insert. Approximately 0.5-1.0 million phage plaques were analyzed per experiment.

Analysis was carried out by transferring the phage DNA of these plaques from the plates onto nitrocellulose filters (0.45 um pore diameter Schleicher and Schuell or Millipore), using standard techniques (Davis and Maniatis). Thus, the DNA pattern on the filter was a replica of the plaque pattern on the plate. After identification of inserts contained within phage DNA that hybridized to the probe, the filters can be matched with the plates and phage isolated.

An oligonucleotide probe, the 48mer, of 48 bases (shown in Figure 2a) was used to screen the random-primed human liver cDNA library H14. The probe corresponded to the molecule spanning the nucleotides encoding amino acids Ala[29] through Leu[44] of the carrier protein subunit S-15. This single oligonucleotide was designed to maximize on bias for human codons.

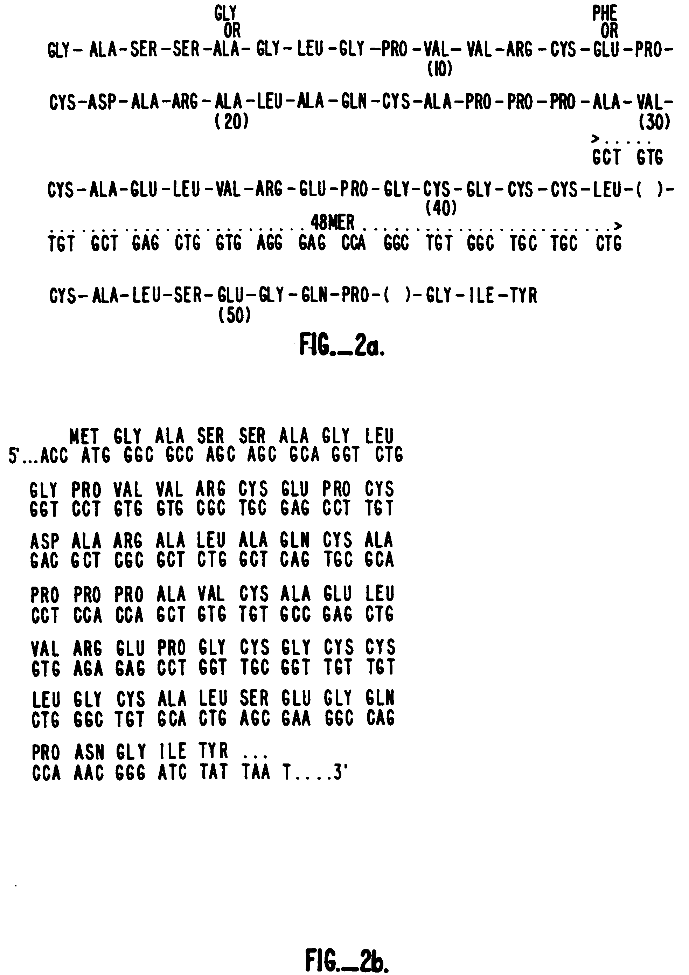

Hybridization conditions were determined by binding the 48-base probe (48mer) to Southern blots of human genomic DNA from the placenta and of a 181-bp synthetic DNA encoding amino acids Gly[1] through Tyr[57] (shown in Figure 2b) under different degrees of stringency. The final conditions for hybridization, which would allow for gene identification with minimal background, was 40% formamide, 5X SSPE (0.9 M sodium chloride, 50 mM sodium phosphate, pH 7.4, 5 mM EDTA), 42°C.

Nitrocellulose filters containing replicas of the phage plaques from the random-primed H14 human liver cDNA library were hybridized with ³²P-labelled 48mer using the hybridization conditions described above. Hybridization was usually carried out overnight, and the filters were rinsed several times in 0.1X SSC (15 mM sodium chloride, 1.5 mM sodium citrate, pH 7.0), 0.1% SDS at 45-50°C prior to autoradiography. DNA's that hybridized strongly to the 4Bmer were identified by autoradiography and the corresponding phage plaques were isolated. Since the original plating of phage was done at a high density, a second round of plating and screening was required to isolate single plaques. This second round of screening also verified that the original isolated phage plaques did indeed hybridize to the 48mer. Single plaques were picked from the plates and the phage were allowed to elute into phage buffer (100 mM NaCl, 10 mM MgSO₄. 50 mM Tris, pH 7.5, 0.01% gelatin). After removing the bacteria by centrifugation, these phage stocks were maintained at 4°C. Phage DNA was purified and characterized (i.e., restricted by restriction endonucleases such as EcoRI in order to determine insert size) following standard procedures (e.g., Maniatis). Inserts were frequently subcloned into smaller plasmids, such as pBR322 or pGEM, at the EcoRI site, using standard procedures.

A number of positive plaques were identified (48 per 600,000 plaques screened). Of these, 9 were chosen for further analysis. Two of these clones (designated cLCP 0.70 and cLCP 0.77), which were approximately 700 to 800 bp in size and which showed the most intense binding by the 48mer probe were cut into smaller fragments prior to sequencing.

Fragments hybridizing to the 48mer, which would be initial sequencing candidates were identified in the following manner. cDNA inserts LCP 0.70 and LCP 0.77 were cleaved with restriction endonuclease HaeIII. These fragments were separated by agarose gel electrophoresis, transferred to a nitrocellulose membrane, and probed with ³²P-labelled 48mer probe. When HaeIII fragments were probed, only one fragment bound the 48mer. This 90 bp fragment was present in both clones LCP 0.70 and LCP 0.77. It was isolated and sequenced according to Sanger, F. et al., Proc. Natl. Acad. Sci., 74, p. 5463 (1977). The DNA molecules of the 90 bp fragments from both LCP 0.70 and LCP 0.77 corresponded exactly to the carrier protein subunit, S-15, amino acid sequence spanning Gln[23] through Glu[50], as shown below. The top line represents the first 57 amino acids of the carrier protein subunit, S-15, and the bottom line represents the translation of the 84 bp HaeIII fragments. The one non-match is the result of the fact that the amino acid at position 45 was unidentified. DNA molecule analysis identified it as a threonine (T).

GASSAGLGPVVRCEPCDARALAQCAPPPAVCAELVREPGCGCCLXCALSEGQPXGIY
                      :::::::::::::::::::::: :::::
                      QCAPPPAVCAELVREPGCGCCLTCALSE

These clones were designated as cLCP 0.70 and cLCP 0.77, their recombinant DNA molecules as λ gtlO:LCP 0.70 and λ gt10:LCP 0.77, and their DNA inserts LCP 0.70 and LCP 0.77. This nomenclature indicates that the clone and recombinant DNA molecule comprises phage λ gt10, containing carrier protein related cDNA isolated from liver cDNA.

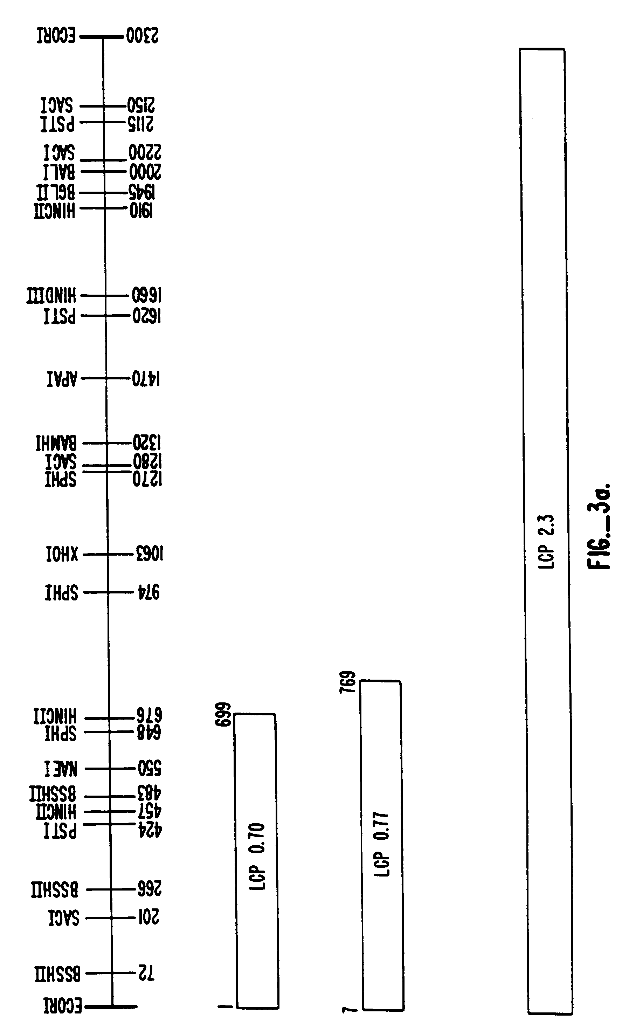

Inserts LCP 0.70 and LCP 0.77 were shown to be similar in size and restriction sites. Inserts LCP 0.70 and LCP 0.77 are approximately 700 and 770 bp, respectively. The restriction maps of LCP 0.70 and LCP 0.77 are shown in Figure 3a. The DNA sequences of the LCP 0.70 and LCP 0.77 inserts, obtained by both single and double-stranded dideoxy-sequencing (Sanger, F., et al., Proc Natl Acad Sci USA 74, 5463 (1977)), are included in the sequence shown in Figure 4, nucleotides 1-699 and 7-769, respectively. In addition to the amino terminus, tryptic fragments T1' and T10 corresponded to the DNA molecules of these clones. LCP 0.70 and LCP 0.77 are sufficiently large to encode 17,558 and 20,320 dalton proteins, respectively. Thus, the information required to encode the entire S-15 molecule is contained within these inserts.

### Identification Of Clones Containing DNA Sequences Coding For Carrier Protein By Cross-Hybridizing To Either LCP 0.70 and LCP 0.77

The recombinant DNA molecules and DNA inserts of clones cLCP 0.70 and cLCP 0.77 isolated as described above, were used to screen the library of clones previously prepared from cDNA by hybridization to phage plaques. This method allows rapid identification of related clones by hybridization of a radioactive probe made from LCP 0.70 to the DNA of recombinant phage fixed on nitrocellulose filters.

Nitrocellulose filters containing phage DNAs that corresponded to phage plaques transferred from LB plates were prepared as described above.

Either the 700 bp LCP 0.70 or the 770 bp LCP 0.77 EcoRI restriction fragment was used to screen human liver random-primed cDNA library H14, human liver oligo-dT-primed cDNA library H10/H14, and human embryonic fibroblast oligo-dT-primed cDNA library WI38. These probes could also be used to screen other cDNA libraries constructed using RNAs from other tissues encoding the carrier protein. In addition they could be used to screen genomic libraries.

The probe fragment (LCP 0.70 or LCP 0.77) was purified by electrophoresis of the EcoRI digestion products of the recombinant DNA molecules (to separate the insert from the cloning vehicle) in about a 1% agarose gel followed by electroelution onto DE81 paper. The specific fragment was then concentrated and ³²P-labelled by "nick translation" by standard procedures.

Hybridization of the above probe to the nitrocellulose filter containing the cDNA clones was carried out essentially as described above.

About 500,000 clones originating from oligo-dT-primed human liver cDNA library H10/H14 and about 500,000 clones originating from oligo-dT-primed human embryonic fibroblast cDNA library WI38 were screened.

The frequency of positive signals in the WI38 fibroblast library was approximately 0.1%, whereas the frequency in the liver libraries was only 0.01-0.02%. Positive clones were plaque-purified and characterized by restriction mapping and sequence analysis to identify other clones containing carrier protein cDNA. Clones were sequenced using single- and double-stranded sequencing techniques (Sanger).

A clone containing a 2.3 kb insert (cLCP 2.3) was isolated from human liver oligo-dT-primed cDNA library H10/H14 which contains that full-length carrier protein-like coding sequence. Clones containing inserts of 1.8 kb (cFCP 1.8) and 2.5 kb (cFCP 2.5), respectively were isolated from the WI38 fibroblast oligo-dT-primed cDNA library. DNA sequence analysis of the clones (Figure 4) showed that both contain the entire carrier protein-like polypeptide coding sequence. The encoded protein consists of a 27 amino acid (81 nucleotide) leader plus a 264 amino acid (792 nucleotide) mature coding region. Both the liver and fibroblast clones display essentially the same nucleotide sequence in the coding region. One of the liver clones encodes a GLY instead of an ALA at amino acid position 5, where position 1 is the first amino acid of the mature protein. This polymorphism corresponds to that observed in carrier protein subunits purified from Cohn fraction IV-1.

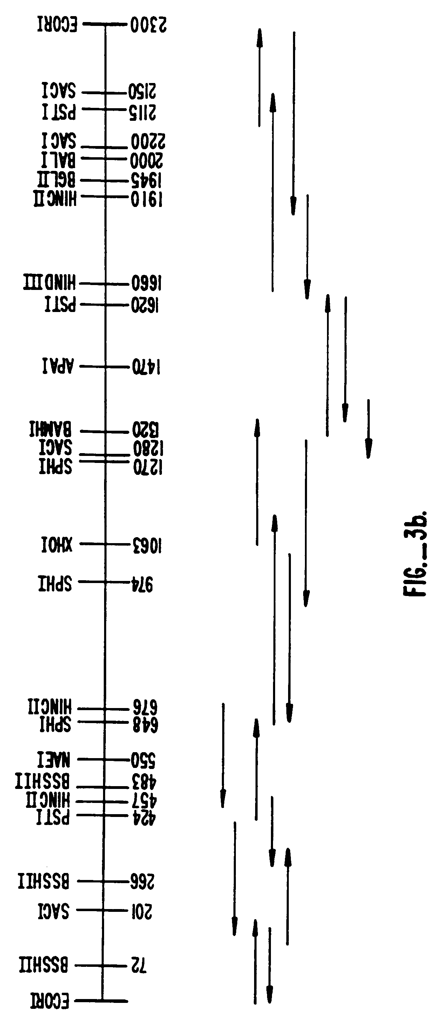

Northern analysis of WI38 human embryonic fibroblast RNA, human liver RNAs H10/H14, human placenta RNA, and macaque liver RNA using LCP 0.70 or LCP 0.77 as a probe indicated that the carrier protein mRNA is approximately 2,000-2,500 bases in size. Thus, the 2.2-2.4 kb clones likely represent full-length cDNAs corresponding to those RNAs. Analysis of the human liver cDNA library and clone cLCP 2.3 by polymerase chain reaction (PCR) amplification (Saiki, R. K., et al. Science 239, pp. 487-491 [1988]) suggests that cLCP 2.3 may have a small deletion of approximately 200 bp in the 3' untranslated region. In fact, recently a clone containing a 2.5 kb insert (cLCP 2.5) was isolated from the liver cDNA library. This insert (LCP 2.5) is the same as LCP 2.3 except for a 200-bp "insertion" between the XhoI site at 1063 and the SphI site at 1270 (Figure 3b). LCP 2.5 is apparently analogous to FCP 2.5.

It is, of course, evident that this method of clone screening using the DNA insert of clones LCP 0.70 and LCP 0.77, as described above, may be employed equally well on other clones containing DNA molecules arising from recombinant DNA technology, synthesis, natural sources or a combination thereof and on clones containing DNA molecules related to any of the above DNA molecules by mutation, including single or multiple, base substitutions, insertions, inversions, or deletions. Therefore, such DNA molecules and their identification also fall within this invention. It is also to be understood that DNA molecules, which are not screened by the above DNA molecule, yet which as a result of their arrangement of nucleotides code for the polypeptides coded for by the above DNA molecules also fall within this invention.

In addition, because of the expected homology between the DNA molecule coding for human carrier protein-like polypeptide and the DNA molecule coding for carrier proteins from non-human sources, the DNA molecules of this invention are useful in the selection of the DNA coding for those non-human carrier proteins and in the cloning and expression of those non-human carrier proteins for use in therapeutic compositions and methods. Finally, the DNA molecules of this invention or oligonucleotides prepared and derived from them may be employed to select other DNA molecules that encode carrier protein-like polypeptides that may not be the carrier protein or a carrier protein subunit. Those molecules and polypeptides are also part of this invention.

### Expression Of Polypeptides Displaying An Activity Of The Carrier Protein

Production of polypeptides by expressing DNA molecules encoding a carrier protein-like polypeptide was carried out in E. coli and mammalian cells.

### Expression in E. coli of Full-Length Carrier Protein-Like Sequence With Alternate Signal Sequence

A DNA fragment containing the entire coding region of the carrier protein gene in which the gene's signal sequence was replaced by that for preproinsulin was ligated into the expression vector pKK233-2 (Pharmacia). This vector contains a trp-lac fusion promoter in which the -35 trp signal is placed 17 bases (the consensus distance) from the lac -10 region. The presence of the lac operator sequences allows expression from this promoter to be induced by adding IPTG (isopropyl-β-D-thiogalactopyranoside) to the medium. In addition, this vector contains the lacZ ribosome binding site.

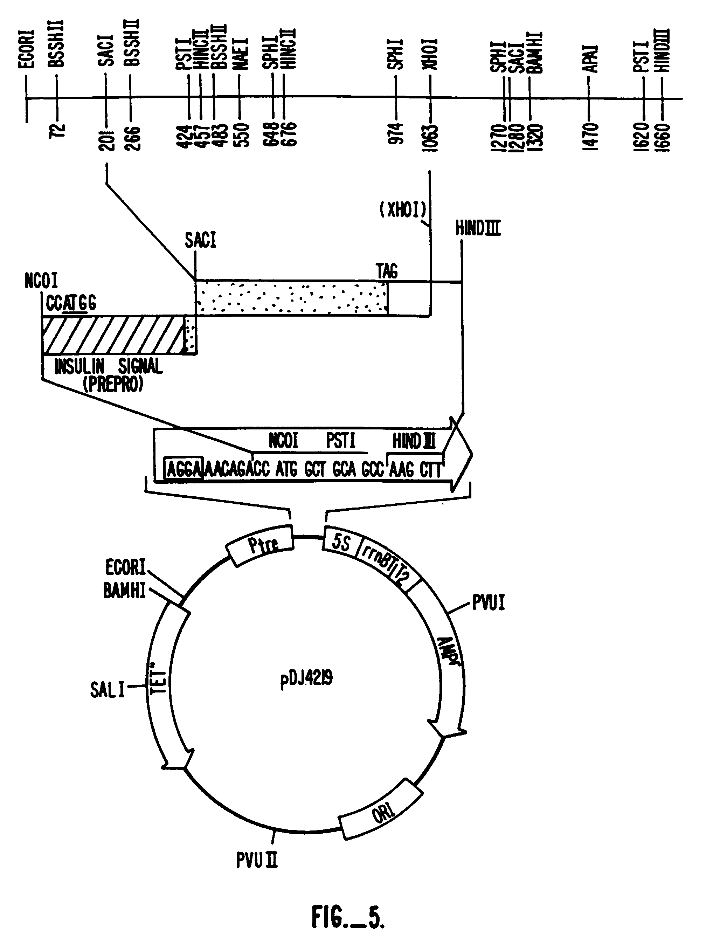

The insert (pDJ4219) containing the preproinsulin signal sequence fused to the carrier protein gene's mature coding sequence was accomplished in the following manner (shown in Figure 5). A preproinsulin signal sequence was synthesized in which the initiating ATG was contained within an NcoI restriction site. The signal sequence was followed by the nucleotides GGCGCGAGCTCG encoding the first four amino acids of the mature carrier protein, through the SacI site. Thus, it was possible to generate the NcoI/SacI fragment shown in Figure 5. This fragment was ligated to the SacI/XhoI fragment containing the rest of the coding sequence for the carrier protein, also shown in Figure 5. The XhoI site, which is located 85 bp beyond the translation termination site, had been converted to a HindIII site by the addition of HindIII linkers using standard procedures. The resulting NcoI/HindIII fragment containing the preproinsulin signal sequence and the carrier protein coding region was inserted into the NcoI and HindIII sites of pKK233-2. Expression of this construction in E. coli induced by IPTG yielded a 25,000-30,000 dalton protein, identified by its ability to bind anti-carrier protein antibody. Expression was carried out in the presence of ³⁵S-cysteine. Two hours after induction by IPTG, the cell extract (cytoplasm and periplasmic space) was immunoprecipitated with anti-carrier protein antibody and submitted to SDS-PAGE. The ability of the carrier protein to be induced by IPTG was demonstrated, since cells containing this construction grown in the absence of IPTG induction expressed only very small quantities of the 25,000-30,000 dalton protein. Controls in which pKK233-2 alone was tested showed no protein in this size range.

### Expression In COS Cells Of A Partial Carrier Protein-Like Sequence

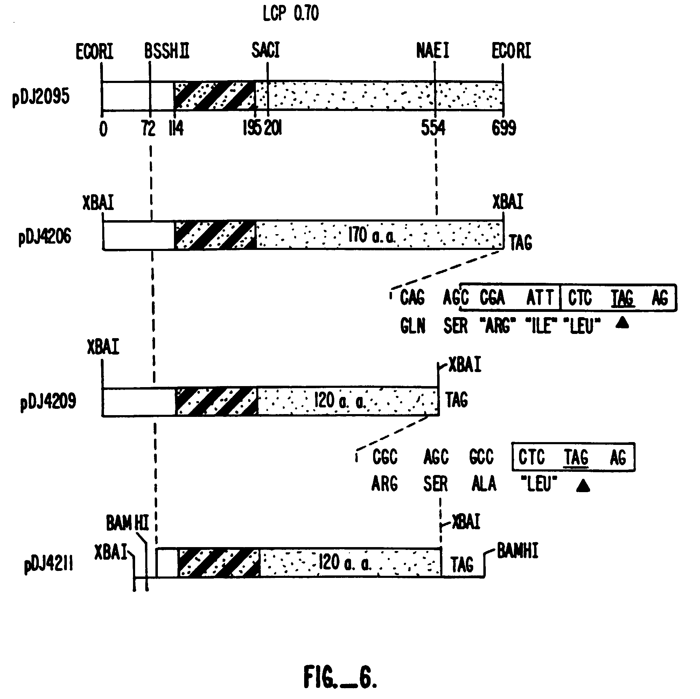

Insert fragments from pDJ4209 and pDJ4211 (shown in Figure 6) were ligated into mammalian expression vector pSVL or pDJ4210 (Pharmacia) at the unique XbaI site. pSVL contains the SV40 late promoter, intron, and polyadenylation site. It also has SV40 and pBR322 origins of replication. pDJ4210 is similar to pSVL but contains the origin of replication from pUC19 instead of pBR322.

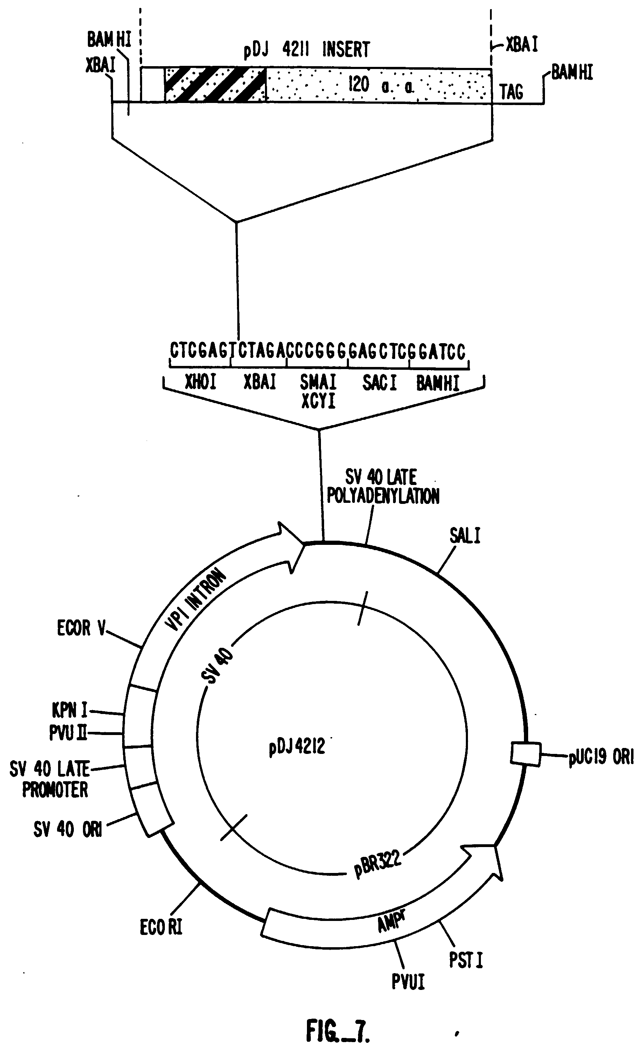

Each of these inserts contains a partial carrier protein gene, specifically the first 120 codons of the mature sequence followed by a synthetic sequence (5'-CTCTAGAG..3') which terminates the reading frame. Each has a different control region:
pDJ4209 contains the entire 5' untranslated region (114 nucleotides) stretching from the EcoRI site, which has been converted to an XbaI site. It also contains the carrier protein signal sequence. The pDJ4209 XbaI fragment contained in pSVL is called pDJ4207.
pDJ4211 contains a 44 nucleotide 5' untranslated region and the carrier protein signal sequence. The pDJ4211 XbaI fragment contained in pDJ4210 is called pDJ4212 and is shown in Figure 7.

The vectors containing the partial carrier protein genes were transfected into COS cells (defective SV40 transformed simian cells) to measure transient expression. Cells were grown in DMEM-F12. Proteins were labelled with ³⁵S-cysteine. Media was collected, immunoprecipitated with anti-carrier protein antibody, and submitted to SDS-PAGE. Expression studies using pDJ4212 and pDJ4207 yielded two proteins of approximately 14,000 and 16,000 daltons. Expression of these proteins was greater with pDJ4212 than with pDJ4207.

### Expression In CHO Cells Of A Full-Length Carrier Protein-Like Sequence

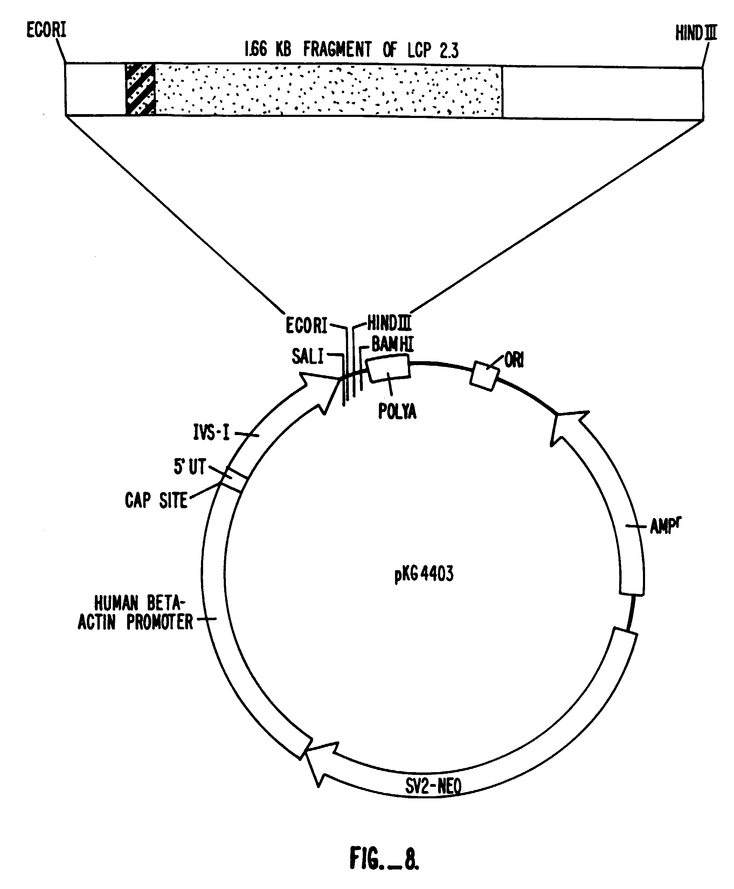

The 1.66 kb EcoRI/HindIII fragment of LCP 2.3 which contains the entire carrier protein gene plus 5' and 3' untranslated regions (114 and 700 nucleotides, respectively) was inserted into mammalian expression vector pKG3226 which contains a β-actin promoter (licensed from Stanford University) and other functions necessary for expression in mammalian cells. The resultant vector, called pKG4403 is shown in Figure 8. pKG4403 was transformed into CHO (Chinese hamster ovary) cells; stably transformed lines were established by drug selection. Serum-free conditioned media from the transformed CHO pool was analyzed for carrier protein-like polypeptide expression by immunoprecipitation of ³⁵S-labelled products and by ability to bind ¹²⁵I-SM-C in an SM-C Western. For detection by immunoprecipitation, cells were grown to 80% confluence in DMEM-F12 supplemented with 10% fetal bovine serum, switched to serum-free media, starved for cysteine 1 hour, and subsequently labelled overnight with ³⁵S-cysteine. The media was immunoprecipitated with anti-carrier protein subunit antibody, and the resulting proteins were analyzed by SDS-PAGE under reducing conditions. Carrier protein-like polypeptides of 37,000 and 39,000 daltons were specifically identified. For detection by SM-C binding, serum) free conditioned media (unlabelled) was collected 48 hours after seeding the transformed pool and was subjected to SDS-PAGE under nonreducing conditions. The proteins were transferred from the gel to a nitrocellulose filter which was probed with ¹²⁵I-SM-C. Two novel carrier protein-like polypeptides of 43,000 and 45,000 daltons were observed. A 23,000 dalton protein endogenous to CHO cells was detected in the transformed pool as well as in the non-transformed control CHO pool. The size difference (37,000 and 39,000 versus 43,000 and 45,000) is likely due to whether SDS-PAGE was conducted under reducing or non-reducing conditions.

This gene of LCP 2.3 does not exclude the possibility that modifications to the gene such as mutations, including single or multiple, base substitutions, deletions, insertions, or inversions may not have already occurred in the gene or may not be employed subsequently to modify its properties or the properties of the polypeptides expressed therefrom. Nor does it exclude any polymorphism which may result in physiologically similar but structurally slightly different genes or polypeptides than that shown in Figure 4.

It should, of course, be understood that cloned cDNA from polyA⁺ RNA by the usual procedures may lack 5'-terminal nucleotides and may even contain artifactual molecules.

The structure of the polypeptide depicted in Figure 4, of course, does not take into account any modifications to the polypeptide caused by its interaction with in vivo enzymes, e.g., glycosylation. Therefore, it must be understood that the amino acid molecule depicted in Figure 4 may not be identical with carrier protein produced in vivo.

It should be understood that while the chromosomal gene encoding carrier protein activity may not be expressible in bacterial hosts because these intervening molecules may not be processed correctly by such hosts, the chromosomal genes are likely to be very useful in the production of carrier protein-like polypeptides in eukaryotic hosts where the human noncoding regions, introns and coding regions may be important for high levels of expression and correct processing of the product to biologically active carrier protein-like polypeptides.

### Improving The Yield And Activity Of Polypeptides Displaying Carrier Protein Activity

The level of production of a protein is governed by three major factors: the number of copies of its gene within the cell, the efficiency with which those gene copies are transcribed and the efficiency with which they are translated. Efficiency of transcription and translation (which together comprise expression) is in turn dependent upon nucleotide molecules, normally situated ahead of the desired coding molecule. These nucleotide molecules or expression control molecules define the location at which RNA polymerase interacts to initiate transcription (the promoter molecule) and at which ribosomes bind and interact with the mRNA (the product of transcription) to initiate translation. Not all such expression control molecules function with equal efficiency. It is thus of advantage to separate the specific coding molecules for the desired protein from their adjacent nucleotide molecules and to fuse them instead to other known expression control molecules so as to favor higher levels of expression. This having been achieved, the newly engineered DNA fragments may be inserted into higher copy number plasmids or bacteriophage derivatives in order to increase the number of gene copies within the cell and thereby further to improve the yield of expressed protein.

Several expression control molecules may by employed as described above. These include the operator, promoter and ribosome binding and interaction molecules (including molecules such as the Shine-Dalgarno molecules) of the lactose operon of E. coli ("the lac system"), the corresponding molecules of the tryptophan synthetase system of E. coli ("the trp system"), the major operator and promoter regions of phage λ (O_{L}P_{L} and O_{R}P_{R}), the bacteriophage T7 promoter recognized only be T7 RNA polymerase, a control region of Filamentous single-stranded DNA phages, SV40 early and late promoters, actin promoters, promoters located on the long terminal repeats of retroviruses, or other molecules which control the expression of genes of prokaryotic or eukaryotic cells and their viruses or combinations thereof. Therefore, to improve the production of a particular polypeptide in an appropriate host, the gene coding for that polypeptide may be prepared as before and inserted into a recombinant DNA molecule closer to its former expression control molecule or under the control of one of the above improved expression control molecules. Such methods are known in the art.

Other methods to improve the efficiency of translation involve insertion of chemically or enzymatically prepared oligonucleotides in front of the initiating codon. By this procedure a more optimal primary and secondary structure of the messenger RNA can be obtained. More specifically, a molecule can be so designed that the initiating AUG codon occurs in a readily accessible position (i.e., not masked by secondary structure) either at the top of a hairpin or in other single-stranded regions. Also the position and molecule of the aforementioned Shine-Dalgarno segment can likewise be optimized. The importance of the general structure (folding) of the messenger RNA has been documented.

Further increases in the cellular yield of the desired products depend upon an increase in the number of genes that can be utilized in the cell. This may be achieved by insertion of the carrier protein-like gene (with or without its transcription and translation control elements) in a higher copy number plasmid or in a temperature-controlled copy number plasmid (i.e., a plasmid which carries a mutation such that the copy number of the plasmid increases after shifting up the temperature.

Alternatively, an increase in gene dosage can be achieved for example by insertion of recombinant DNA molecules engineered in the way described previously into the temperate bacteriophage, most simply by digestion of the plasmid with a restriction enzyme, to give a linear molecule which is then mixed with a restricted phage λ cloning vehicle and the recombinant DNA molecule produced by incubation with DNA ligase. The desired recombinant phage is then selected as before and used to lysogenize a host strain of E. coli.

Therefore, it should be understood that the insert DNA of this invention may be inserted into other expression vectors, as previously described (supra) and these vectors employed in various hosts, as previously described (supra) to improve the expression of the gene coding for carrier protein subunit.

The biological activity of the carrier protein-like polypeptides produced in accordance with this invention may also be improved by using the DNA molecules of this invention to transform mammalian cell systems and to express the gene in those systems. Such mammalian systems are known. One such system is the CHO (Chinese Hamster ovary) (DHFR⁻) cell system in which the gene expression may be amplified by methotrexate (MTX). These expression systems permit the production of glycosylated proteins. Such cells can be induced to greatly amplify the copy number of the carrier protein-like gene.

It should also be understood that carrier protein-like polypeptides may also be prepared in the form of a fused protein (e.g., linked to a prokaryotic or eukaryotic N-terminal segment directing excretion), in the form of procarrier protein-like polypeptide (e.g., starting with all or parts of the carrier protein signal molecule which could be cleaved off upon excretion) or as a mature carrier protein-like polypeptide (by cleavage of any extraneous amino acids, including an initial methionine during expression and excretion) or in the form of a f-met-carrier protein-like polypeptide. One particularly useful polypeptide in accordance with this invention would be mature carrier-like polypeptide with an easily cleaved amino acid or series of amino acids attached to the amino terminus. Such constructions would allow synthesis of the protein in an appropriate host, where a start signal not present in mature carrier protein subunits is needed, and then cleavage of the extra amino acids to produce mature carrier protein subunits.

When the carrier protein subunit or carrier protein-like polypeptide is to be used in combination with somatomedin-like molecules for therapy, the two molecules could be co-produced in the same cell, preferably in mammalian cells. Vectors containing both genes could be cotransformed and stable cell lines selected that expressed both proteins. Thus, only one fermentation and purification scheme would be required to produce the complex containing both carrier protein-like and the somatomedin-like polypeptides.

The yield of these different forms of polypeptide may be improved by any or a combination of the procedures discussed above. Also different codons for some or all of the codons used in the present DNA molecules could be substituted. These substituted codons may code for amino acids identical to those coded for by the codons replaced but result in higher yield of the polypeptide. Alternatively, the replacement of one or a combination of codons leading to amino acid replacement or to a longer or shorter carrier protein-like polypeptide may alter its properties in a useful way (e.g., increase the stability, increase the solubility, increase the therapeutic activity).

Finally, the activity of the polypeptides produced by the recombinant DNA molecules of this invention may be improved by fragmenting, modifying or derivatizing the DNA molecules or polypeptides of this invention by well-known means, without departing from the scope of this invention.

While we have described certain embodiments of the invention, it is apparent that those embodiments can be altered to provide other embodiments which utilize the processes and compositions of the invention. The scope of the invention is defined by the following claims rather than by the specific embodiments that have been presented by way of example.

## Claims (Claims for the following Contracting State(s): AT, BE, CH, LI, DE, FR, GB, IT, LU, NL, SE)

1. A carrier protein-like polypeptide which comprises a sequence having an in-vivo effector activity of a polypeptide with the sequence of Fig. 4 and lacking the natural glycosylation of the carrier protein.

2. A polypeptide of claim 1 with the sequence of Figure 4.

3. A polypeptide of claim 1 or claim 2 being essentially free of substances naturally present in human serum.

4. An essentially pure polypeptide having the sequence of amino acids 27 to 290 of Figure 4 and having a methionine residue preceding amino acid 27.

5. The polypeptide of claim 4 wherein one or more amino acid residues have been added, deleted or substituted and the polypeptide constitutes a carrier protein-like polypeptide.

6. A polypeptide selected from the group consisting of a polypeptide having the sequence of amino acids -1 to 290 of Figure 4, and polypeptides having a portion of that sequence and constituting a carrier protein-like polypeptide.

7. A polypeptide according to any one of claims 1 to 6 substantially complexed with at least one somatomedinlike polypeptide.

8. A therapeutic composition comprising an effective amount of a polypeptide according to any one of claims 1 to 6 for inhibiting the growth of keloid scars and for inhibiting the growth of somatomedin-dependent cancers.

9. A therapeutic composition comprising an effective amount of a polypeptide according to any one of claims 1 to 6 substantially complexed with at least one somatomedin-like polypeptide for treating osteoporosis in humans.

10. Use of a polypeptide according to any one of claims 1 to 6 in the manufacture of a medicament for inhibiting the growth of keloid scars or in the manufacture of a medicament for inhibiting the growth of somatomedin-dependent cancers.

11. Use of a polypeptide according to claim 6 in the manufacture of a medicament for the treatment of osteoporosis.

12. A DNA isolate which comprises a DNA sequence which codes for a polypeptide having the sequence of amino acids -1 to 290 of Figure 4.

13. A DNA isolate which comprises a DNA sequence which codes for a polypeptide having the sequence of amino acids 27 to 290 of Figure 4.

14. A DNA isolate which comprises a DNA sequence which codes for a polypeptide having the sequence of amino acids 27 to 290 of Figure 4 and having a methionine residue preceding amino acid 27.

15. A recombinant DNA molecule which comprises a DNA sequence as defined in any one of claims 12 to 14 operatively linked to an expression control sequence.

16. A host cell transformed with recombinant DNA molecule according to claim 15.

17. A method for producing a carrier protein-like polypeptide, comprising culturing a host cell according to claim 16 to make said polypeptide.

18. A vector comprising a recombinant DNA molecule according to claim 15.

19. A host transformed with a vector according to claim 18.

20. A method for producing a carrier protein-like polypeptide which comprises culturing a host transformed by a vector according to claim 18 to make said polypeptide.

## Claims (Claims for the following Contracting State(s): ES)

1. A process which comprises the preparation of a carrier protein-like polypeptide which comprises a sequence having an in-vivo effector activity of a polypeptide with the sequence of Fig. 4 and lacking the natural glycosylation of the carrier protein.

2. A process according to claim 1, wherein the polypeptide has the sequence of Figure 4.

3. A process according to claim 1 or 2, wherein the polypeptide is essentially free of substances naturally present in human serum.

4. A process which comprises the preparation of an essentially pure polypeptide having the sequence of amino acids 27 to 290 of Figure 4 and having a methionine residue preceding amino acid 27.

5. A process according to claim 4 wherein the polypeptide has one or more amino acid residues added, deleted or substituted and constitutes a carrier protein-like polypeptide.

6. A process which comprises the preparation of a polypeptide selected from the group consisting of polypeptides having the sequence of amino acids -1 to 290 of Figure 4, and polypeptides having a portion of that sequence and constituting a carrier protein-like polypeptide.

7. A process according to any one of claims 1 to 6, wherein the polypeptide is substantially complexed with at least one somatomedin-like polypeptide.

8. A process which comprises the preparation of a therapeutic composition which comprises an effective amount of a polypeptide as defined in any one of claims 1 to 6 for inhibiting the growth of keloid scars and for inhibiting the growth of somatomedin-dependent cancers.

9. A process which comprises the preparation of a therapeutic composition which comprises an effective amount of a polypeptide as defined in any one of claims 1 to 6 substantially complexed with at least one somatomedin-like polypeptide for treating osteoporosis in humans.

10. Use of a polypeptide as defined in any one of claims 1 to 6 in the manufacture of a medicament for inhibiting the growth of keloid scars or in the manufacture of a medicament for inhibiting the growth of somatomedin-dependent cancers.

11. Use of a polypeptide as defined in claim 6 in the manufacture of a medicament for the treatment of osteoporosis.

12. A process which comprises the preparation of a DNA isolate which comprises a DNA sequence which codes for a polypeptide having the sequence of amino acids -1 to 290 of Figure 4.

13. A process which comprises the preparation of a DNA isolate which comprises a DNA sequence which codes for a polypeptide having the sequence of amino acids 27 to 290 of Figure 4.

14. A process which comprises the preparation of a DNA isolate which comprises a DNA sequence which codes for a polypeptide having the sequence of amino acids 27 to 290 of Figure 4 and having a methionine residue preceding amino acid 27.

15. A process which comprises the preparation of a recombinant DNA molecule which comprises a DNA sequence as defined in any one of claims 12 to 14 operatively linked to an expression control sequence.

16. A host cell transformed with a recombinant DNA molecule as defined in claim 15.

17. A process for producing a carrier protein-like polypeptide which comprises culturing a host cell according to claim 16 to make said polypeptide.

18. A process which comprises the preparation of a vector comprising a recombinant DNA molecule as defined in claim 15.

19. A vector comprising a recombinant DNA molecule as defined in claim 15.

20. A host cell transformed with vector as defined in claim 18.

21. A process for producing a carrier protein-like polypeptide which comprises culturing a host transformed by a vector as defined in claim 18 to make said polypeptide.

## Claims (Claims for the following Contracting State(s): GR)

1. A carrier protein-like polypeptide which comprises a sequence having an in-vivo effector activity of a polypeptide with the sequence of Fig. 4 and lacking the natural glycosylation of the carrier protein.

2. A polypeptide of claim 1 with the sequence of Figure 4.

3. A polypeptide of claim 1 or claim 2 being essentially free of substances naturally present in human serum.

4. An essentially pure polypeptide having the sequence of amino acids 27 to 290 of Figure 4 and having a methionine residue preceding amino acid 27.

5. The polypeptide of claim 4 wherein one or more amino acid residues have been added, deleted or substituted and the polypeptide constitutes a carrier protein-like polypeptide.

6. A polypeptide selected from the group consisting of a polypeptide having the sequence of amino acids -1 to 290 of Figure 4, and polypeptides having a portion of that sequence and constituting a carrier protein-like polypeptide.

7. A polypeptide according to any one of claims 1 to 6 substantially complexed with at least one somatomedin-like polypeptide.

8. A process which comprises the preparation of a therapeutic composition which comprises an effective amount of a polypeptide as defined in any one of claims 1 to 6 for inhibiting the growth of keloid scars and for inhibiting the growth of somatomedin-dependent cancers.

9. A process which comprises the preparation of a therapeutic composition which comprises an effective amount of a polypeptide according to any one of claims 1 to 6 substantially complexed with at least one somatomedin-like polypeptide for treating osteoporosis in humans.

10. Use of a polypeptide according to any one of claims 1 to 6 in the manufacture of a medicament for inhibiting the growth of keloid scars or in the manufacture of a medicament for inhibiting the growth of somatomedin-dependent cancers.

11. Use of a polypeptide according to claim 6 in the manufacture of a medicament for the treatment of osteoporosis.

12. A DNA isolate which comprises a DNA sequence which codes for a polypeptide having the sequence of amino acids -1 to 290 of Figure 4.

13. A DNA isolate which comprises a DNA sequence which codes for a polypeptide having the sequence of amino acids 27 to 290 of Figure 4.

14. A DNA isolate which comprises a DNA sequence which codes for a polypeptide having the sequence of amino acids 27 to 290 of Figure 4 and having a methionine residue preceding amino acid 27.

15. A recombinant DNA molecule which comprises a DNA sequence as defined in any one of claims 12 to 14 operatively linked to an expression control sequence.

16. A host cell transformed with recombinant DNA molecule according to claim 15.

17. A method for producing a carrier protein-like polypeptide, comprising culturing a host cell according to claim 16 to make said polypeptide.

18. A vector comprising a recombinant DNA molecule according to claim 15.

19. A host transformed with a vector according to claim 18.

20. A method for producing a carrier protein-like polypeptide which comprises culturing a host transformed by a vector according to claim 18 to make said polypeptide.

## Patentansprüche (Patentansprüche für folgende(n) Vertragsstaat(en): AT, BE, CH, LI, DE, FR, GB, IT, LU, NL, SE)

1. Trägerproteinartiges Polypeptid, das eine Sequenz umfaßt, die die in-vivo-Effektoraktivität eines Polypeptids mit der Sequenz von Fig. 4 aufweist und der die natürliche Glykosylierung des Trägerproteins fehlt.

2. Polypeptid nach Anspruch 1 mit der Sequenz von Fig. 4.

3. Polypeptid nach Anspruch 1 oder 2, das im wesentlichen frei von Substanzen ist, die in menschlichem Serum auf natürliche Weise vorhanden sind.

4. Im wesentlichen reines Polypeptid, das die Sequenz der Aminosäuren 27 bis 290 von Fig. 4 aufweist, bei dem der Aminosäure 27 ein Methioninrest vorangeht.

5. Polypeptid nach Anspruch 4, worin ein oder mehrere Aminosäurereste hinzugefügt, gelöscht oder substituiert worden sind und worin das Polypeptid ein trägerproteinartiges Polypeptid darstellt.

6. Polypeptid, das aus der Gruppe ausgewählt ist, die aus einem Polypeptid mit der Sequenz der Aminosäuren -1 bis 290 von Fig. 4 und Polypeptiden, die einen Abschnitt dieser Sequenz aufweisen und ein trägerproteinartiges Polypeptid darstellen, besteht.

7. Polypeptid nach einem der Ansprüche 1 bis 6, das im wesentlichen mit zumindest einem somatomedinartigen Polypeptid komplexiert ist.

8. Therapeutische Zusammensetzung, die eine wirksame Menge eines Polypeptids nach einem der Ansprüche 1 bis 6 zur Hemmung des Wachstums von Narbengeschwulsten oder zur Hemmung des Wachstums von somatomedinabhängigen Krebsarten umfaßt.

9. Therapeutische Zusammensetzung, die eine wirksame Menge eines Polypeptids nach einem der Ansprüche 1 bis 6, das im wesentlichen mit zumindest einem somatomedinartigen Polypeptid komplexiert ist, umfaßt, zur Behandlung von Osteoporose bei Menschen.

10. Verwendung eines Polypeptids nach einem der Ansprüche 1 bis 6 bei der Herstellung eines Medikaments zur Hemmung des Wachstums von Narbengeschwulsten oder bei der Herstellung eines Medikaments zur Hemmung des Wachstums von somatomedinabhängigen Krebsarten.

11. Verwendung eines Polypeptids nach Anspruch 6 bei der Herstellung eines Medikaments zur Behandlung von Osteoporose.

12. DNA-lsolat, das eine DNA-Sequenz umfaßt, die für ein Polypeptid mit der Sequenz der Aminosäuren -1 bis 290 von Fig. 4 kodiert.

13. DNA-lsolat, das eine DNA-Sequenz umfaßt, die für ein Polypeptid mit der Sequenz der Aminosäuren 27 bis 290 von Fig. 4 kodiert.

14. DNA-lsolat, das eine DNA-Sequenz umfaßt, die für ein Polypeptid mit der Sequenz der Aminosäuren 27 bis 290 von Fig. 4 kodiert und bei der der Aminosäure 27 ein Methioninrest vorangeht.

15. Rekombinantes DNA-Molekül, das eine nach einem der Ansprüche 12 bis 14 definierte DNA-Sequenz umfaßt, die operativ mit einer Expressionssteuerungssequenz verbunden ist.

16. Wirtszelle, die mit einem rekombinanten DNA-Molekül nach Anspruch 15 transformiert ist.

17. Verfahren zur Herstellung eines trägerproteinartigen Polypeptids, umfassend das Kultivieren einer Wirtszelle nach Anspruch 16 zur Herstellung des Polypeptids.

18. Vektor, umfassend ein rekombinantes DNA-Molekül nach Anspruch 15.

19. Wirt, der mit einem Vektor nach Anspruch 18 transformiert ist.

20. Verfahren zur Herstellung eines trägerproteinartigen Polypeptids, welches das Kultivieren eines mit einem Vektor nach Anspruch 18 transformierten Wirts umfaßt, um das Polypeptid herzustellen.

## Patentansprüche (Patentansprüche für folgende(n) Vertragsstaat(en): ES)

1. Verfahren, welches die Herstellung eines trägerproteinartigen Polypeptids umfaßt, das eine Sequenz umfaßt, die die in-vivo-Effektoraktivität eines Polypeptids mit der Sequenz von Fig. 4 aufweist und der die natürliche Glykosylierung des Trägerproteins fehlt.

2. Verfahren nach Anspruch 1, worin das Polypeptid die Sequenz von Fig. 4 hat.

3. Verfahren nach Anspruch 1 oder 2, worin das Polypeptid im wesentlichen frei von Substanzen ist, die in menschlichem Serum auf natürliche Weise vorhanden sind.

4. Verfahren, das die Herstellung eines im wesentlichen reinen Polypeptids, das die Sequenz der Aminosäuren 27 bis 290 von Fig. 4 aufweist und bei dem der Aminosäure 27 ein Methioninrest vorangeht.

5. Verfahren nach Anspruch 4, worin im Polypeptid ein oder mehrere Aminosäurereste hinzugefügt, gelöscht oder substituiert worden sind und worin das Polypeptid ein trägerproteinartiges Polypeptid darstellt.

6. Verfahren, welches die Herstellung eines Polypeptids umfaßt, das aus der Gruppe ausgewählt ist, die aus Polypeptiden mit der Sequenz der Aminosäuren -1 bis 290 von Fig. 4 und Polypeptiden, die einen Abschnitt dieser Sequenz aufweisen und ein trägerproteinartiges Polypeptid darstellen, besteht.

7. Verfahren nach einem der Ansprüche 1 bis 6, worin das Polypeptid im wesentlichen mit zumindest einem somatomedinartigen Polypeptid komplexiert ist.

8. Verfahren, welches die Herstellung einer therapeutischen Zusammensetzung umfaßt, die eine wirksame Menge eines nach einem der Ansprüche 1 bis 6 definierten Polypeptids zur Hemmung des Wachstums von Narbengeschwulsten oder zur Hemmung des Wachstums von somatomedinabhängigen Krebsarten umfaßt.

9. Verfahren, welches die Herstellung einer therapeutischen Zusammensetzung umfaßt, die eine wirksame Menge eines nach einem der Ansprüche 1 bis 6 definierten Polypeptids, das im wesentlichen mit zumindest einem somatomedinartigen Polypeptid komplexiert ist, umfaßt, zur Behandlung von Osteoporose bei Menschen.

10. Verwendung eines Polypeptids nach einem der Ansprüche 1 bis 6 bei der Herstellung eines Medikaments zur Hemmung des Wachstums von Narbengeschwulsten oder bei der Herstellung eines Medikaments zur Hemmung des Wachstums von somatomedinabhängigen Krebsarten.

11. Verwendung eines Polypeptids nach Anspruch 6 bei der Herstellung eines Medikaments zur Behandlung von Osteoporose.

12. Verfahren, welches die Herstellung eines DNA-lsolats umfaßt, das eine DNA-Sequenz umfaßt, die für ein Polypeptid mit der Sequenz der Aminosäuren -1 bis 290 von Fig. 4 kodiert.

13. Verfahren, welches die Herstellung eines DNA-lsolats umfaßt, das eine DNA-Sequenz umfaßt, die für eine Polypeptid mit der Sequenz der Aminosäuren 27 bis 290 von Fig. 4 kodiert.

14. Verfahren, welches die Herstellung eines DNA-lsolats umfaßt, das eine DNA-Sequenz umfaßt, die für ein Polypeptid mit der Sequenz der Aminosäuren 27 bis 290 von Fig. 4 kodiert und bei der der Aminosäure 27 ein Methioninrest vorangeht.

15. Verfahren, welches die Herstellung eines rekombinanten DNA-Moleküls umfaßt, das eine nach einem der Ansprüche 12 bis 14 definierte DNA-Sequenz umfaßt, die operativ mit einer Expressionssteuerungssequenz verbunden ist.

16. Wirtszelle, die mit einem rekombinanten DNA-Molekül nach Anspruch 15 transformiert ist.

17. Verfahren zur Herstellung eines trägerproteinartigen Polypeptids, umfassend das Kultivieren einer Wirtszelle nach Anspruch 16 zur Herstellung des Polypeptids.

18. Verfahren, das die Herstellung eines Vektors umfaßt, der ein rekombinantes DNA-Molekül nach Anspruch 15 umfaßt.

19. Vektor, der ein rekombinantes DNA-Molekül nach Anspruch 15 umfaßt.

20. Wirtszelle, die mit einem Vektor nach Anspruch 19 transformiert ist.

21. Verfahren zur Herstellung eines trägerproteinartigen Polypeptids, welches das Kultivieren eines mit einem Vektor nach Anspruch 19 transformierten Wirts umfaßt, um das Polypeptid herzustellen.

## Patentansprüche (Patentansprüche für folgende(n) Vertragsstaat(en): GR)

1. Trägerproteinartiges Polypeptid, das eine Sequenz umfaßt, die die in-vivo-Effektoraktivität eines Polypeptids mit der Sequenz von Fig. 4 aufweist und der die natürliche Glykosylierung des Trägerproteins fehlt.

2. Polypeptid nach Anspruch 1 mit der Sequenz von Fig. 4.

3. Polypeptid nach Anspruch 1 oder 2, das im wesentlichen frei von Substanzen ist, die in menschlichem Serum auf natürliche Weise vorhanden sind.

4. Im wesentlichen reines Polypeptid mit der Sequenz der Aminosäuren 27 bis 290 von Fig. 4, bei dem Aminosäure 27 ein Methioninrest vorangeht.

5. Polypeptid nach Anspruch 4, worin ein oder mehrere Aminosäurereste hinzugefügt, gelöscht oder substituiert worden sind und worin das Polypeptid ein trägerproteinartiges Polypeptid darstellt.

6. Polypeptid, das aus der Gruppe ausgewählt ist, die aus einem Polypeptid mit der Sequenz der Aminosäuren -1 bis 290 von Fig. 4 und Polypeptiden, die einen Abschnitt dieser Sequenz aufweisen und ein trägerproteinartiges Polypeptid darstellen, besteht.

7. Polypeptid nach einem der Ansprüche 1 bis 6, das im wesentlichen mit zumindest einem somatomedinartigen Polypeptid komplexiert ist.

8. Verfahren, welches die Herstellung einer therapeutischen Zusammensetzung umfaßt, die eine wirksame Menge eines Polypeptids nach einem der Ansprüche 1 bis 6 zur Hemmung des Wachstums von Narbengeschwulsten oder zur Hemmung des Wachstums von somatomedinabhängigen Krebsarten umfaßt.

9. Verfahren, welches die Herstellung einer therapeutischen Zusammensetzung umfaßt, die eine wirksame Menge eines Polypeptids nach einem der Ansprüche 1 bis 6, das im wesentlichen mit zumindest einem somatomedinartigen Polypeptid komplexiert ist, umfaßt, zur Behandlung von Osteoporose bei Menschen.

10. Verwendung eines Polypeptids nach einem der Ansprüche 1 bis 6 bei der Herstellung eines Medikaments zur Hemmung des Wachstums von Narbengeschwulsten oder bei der Herstellung eines Medikaments zur Hemmung des Wachstums von somatomedinabhängigen Krebsarten.

11. Verwendung eines Polypeptids nach Anspruch 6 bei der Herstellung eines Medikaments zur Behandlung von Osteoporose.

12. DNA-Isolat, das eine DNA-Sequenz umfaßt, die für ein Polypeptid mit der Sequenz der Aminosäuren -1 bis 290 von Fig. 4 kodiert.

13. DNA-lsolat, das eine DNA-Sequenz umfaßt, die für eine Polypeptid mit der Sequenz der Aminosäuren 27 bis 290 von Fig. 4 kodiert.

14. DNA-lsolat, das eine DNA-Sequenz umfaßt, die für ein Polypeptid mit der Sequenz der Aminosäuren 27 bis 290 von Fig. 4 kodiert und bei der der Aminosäure 27 ein Methioninrest vorangeht.

15. Rekombinantes DNA-Molekül, das eine nach einem der Ansprüche 12 bis 14 definierte DNA-Sequenz umfaßt, die operativ mit einer Expressionssteuerungssequenz verbunden ist.

16. Wirtszelle, die mit rekombinantem DNA-Molekül nach Anspruch 15 transformiert ist.

17. Verfahren zur Herstellung eines trägerproteinartigen Polypeptids, umfassend das Kultivieren einer Wirtszelle nach Anspruch 16 zur Herstellung des Polypeptids.

18. Vektor, umfassend ein rekombinantes DNA-Molekül nach Anspruch 15.

19. Wirt, der mit einem Vektor nach Anspruch 18 transformiert ist.

20. Verfahren zur Herstellung eines trägerproteinartigen Polypeptids, welches das Kultivieren eines mit einem Vektor nach Anspruch 18 transformierten Wirts umfaßt, um das Polypeptid herzustellen.

## Revendications (Revendications pour l'(les) Etat(s) contractant(s) suivant(s): AT, BE, CH, LI, DE, FR, GB, IT, LU, NL, SE)

1. Polypeptide analogue à une protéine porteuse qui comprend une séquence ayant une activité d'effecteur in-vivo d'un polypeptide avec la séquence de la Figure 4 et n'ayant pas la glycosylation naturelle de la protéine porteuse.

2. Polypeptide selon la revendication 1 avec la séquence de la Figure 4.

3. Polypeptide selon la revendication 1 ou la revendication 2 qui est substantiellement exempt de substances naturellement présentes dans le sérum humain.

4. Polypeptide essentiellement pur ayant la séquence des acides aminés 27 à 290 de la Figure 4 et ayant un résidu méthionine précédant l'acide aminé 27.

5. Polypeptide selon la revendication 4, dans lequel un ou plus résidus acides aminés ont été ajoutés, effacés ou substitués et le polypeptide constitue un polypeptide analogue à une protéine porteuse.

6. Polypeptide choisi dans le groupe consistant en un polypeptide ayant la séquence des acides aminés -1 à 290 de la Figure 4, et polypeptides ayant une portion de cette séquence et constituant un polypeptide analogue à une protéine porteuse.

7. Polypeptide selon l'une quelconque des revendications 1 à 6 substantiellement complexé avec au moins un polypeptide analogue à la somatomédine.

8. Composition thérapeutique comprenant une quantité efficace d'un polypeptide selon l'une quelconque des revendications 1 à 6 pour inhiber la croissance des cicatrices chéloïdiennes et pour inhiber la croissance des cancers dépendant de la somatomédine.

9. Composition thérapeutique comprenant une quantité efficace d'un polypeptide selon l'une quelconque des revendications 1 à 6 complexé substantiellement avec au moins un polypeptide analogue à la somatomédine pour traiter l'ostéoporose chez les humains.

10. Utilisation d'un peptide selon l'une quelconque des revendications 1 à 6 dans la fabrication d'un médicament pour inhiber la croissance des cicatrices chéloïdiennes ou dans la fabrication d'un médicament pour inhiber la croissance des cancers dépendant de la somatomédine.

11. Utilisation d'un polypeptide selon la revendication 6 dans la fabrication d'un médicament pour le traitement de l'ostéoporose.

12. Isolat d'ADN qui comprend une séquence ADN qui code pour un polypeptide ayant la séquence des acides aminés -1 à 290 de la Figure 4.

13. Isolat d'ADN qui comprend une séquence d'ADN qui code pour un polypeptide ayant la séquence des acides aminés 27 à 290 de la Figure 4.

14. Isolat d'ADN qui comprend une séquence d'ADN qui code pour un polypeptide ayant la séquence des acides aminés 27 à 290 de la Figure 4 et ayant un résidu méthionine précédant l'acide aminé 27.

15. Molécule d'ADN recombinant qui comprend une séquence d'ADN telle que définie dans l'une quelconque des revendications 12 à 14 pontée opérationnellement à une séquence de contrôle d'expression.

16. Cellule hôte transformé avec la molécule d'ADN recombinant selon la revendication 15.

17. Méthode pour produire un polypeptide analogue à une protéine porteuse, comprenant la culture d'une cellule hôte selon la revendication 16 pour fabriquer ledit polypeptide.

18. Vecteur comprenant une molécule d'ADN recombinant selon la revendication 15.

19. Hôte transformée avec un vecteur selon la revendication 18.

20. Méthode pour produire un polypeptide analogue à une protéine porteuse qui comprend la culture d'un hôte transformé par un vecteur selon la revendication 18 pour fabriquer ledit polypeptide.

## Revendications (Revendications pour l'(les) Etat(s) contractant(s) suivant(s): ES)

1. Procédé qui comprend la préparation d'un polypeptide analogue à une protéine porteuse qui comprend une séquence ayant une activité d'effecteur in-vivo d'un polypeptide avec la séquence de la Figure 4 et n'ayant pas la glycosylation naturelle de la protéine porteuse.

2. Procédé selon la revendication 1, dans lequel le polypeptide a la séquence de la Figure 4.

3. Procédé selon la revendication 1 ou 2, dans lequel le polypeptide est essentiellement exempt de substances naturellement présentes dans le sérum humain.

4. Procédé qui comprend la préparation d'un polypeptide essentiellement pur ayant la séquence des acides aminés 27 à 290 de la Figure 4 et ayant un résidu méthionine précédant l'acide aminé 27.

5. Procédé selon la revendication 4 dans lequel le polypeptide a un ou plus résidus acides aminés ajoutés, effacés ou substitués et constitue un polypeptide analogue à une protéine porteuse.

6. Procédé qui comprend la préparation d'un polypeptide sélectionné dans le groupe consistant en des polypeptides ayant la séquence des acides aminés -1 à 290 de la Figure 4, et des polypeptides ayant une portion de cette séquence et constituant un polypeptide analogue à une protéine porteuse.

7. Procédé selon l'une quelconque des revendications 1 à 6, dans lequel le polypeptide est substantiellement complexé avec au moins un polypeptide analogue à la somatomédine.

8. Procédé qui comprend la préparation d'une composition thérapeutique qui comprend une quantité efficace d'un polypeptide tel que défini dans l'une quelconque des revendications 1 à 6 pour inhiber la croissance des cicatrices chéloïdiennes et pour inhiber la croissance des cancers dépendant de la somatomédine.

9. Procédé qui comprend la préparation d'une composition thérapeutique qui comprend une quantité efficace d'un polypeptide tel que défini dans l'une quelconque des revendications 1 à 6 complexé substantiellement avec au moins un polypeptide analogue à la somatomédine pour traiter l'ostéoporose chez les humains.

10. Utilisation d'un polypeptide tel que défini dans l'une quelconque des revendications 1 à 6 dans la fabrication d'un médicament pour inhiber la croissance des cicatrices chéloïdiennes ou dans la fabrication de médicaments pour inhiber la croissance des cancers dépendant de la somatomédine.

11. Utilisation d'un polypeptide selon la revendication 6 dans la fabrication d'un médicament pour le traitement de l'ostéoporose.

12. Procédé qui comprend la préparation d'un isolat d'ADN qui comprend une séquence d'ADN qui code pour un polypeptide ayant la séquence des acides aminés -1 à 290 de la Figure 4.

13. Procédé qui comprend la préparation d'un isolat d'ADN qui comprend une séquence d'ADN qui code pour un polypeptide ayant la séquence des acides aminés 27 à 290 de la Figure 4.

14. Procédé qui comprend la préparation d'un isolat d'ADN qui comprend une séquence d'ADN qui code pour un polypeptide ayant la séquence des acides aminés 27 à 290 de la Figure 4 ayant un résidu méthionine précédant l'acide aminé 27.

15. Procédé qui comprend la préparation d'une molécule d'ADN recombinante qui comprend une séquence d'ADN selon l'une quelconque des revendications 12 à 14 pontée opérationnellement à une séquence de contrôle d'expression.

16. Cellule hôte transformée avec une molécule d'ADN recombinant telle que définie dans la revendication 15.

17. Procédé pour produire un polypeptide analogue à une protéine porteuse qui comprend la culture d'une cellule hôte selon la revendication 16 pour fabriquer ledit polypeptide.

18. Procédé qui comprend la préparation d'un vecteur comprenant une molécule d'ADN recombinant telle que définie dans la revendication 15.

19. Vecteur comprenant une molécule d'ADN recombinant telle que définie dans la revendication 15.

20. Cellule hôte transformée avec un vecteur tel que défini dans la revendication 18.

21. Procédé pour produire un polypeptide analogue à une protéine porteuse qui comprend la culture d'un hôte transformé par un vecteur tel que défini dans la revendication 18 pour fabriquer ledit polypeptide.

## Revendications (Revendications pour l'(les) Etat(s) contractant(s) suivant(s): GR)

1. Polypeptide analogue à une protéine porteuse qui comprend une séquence ayant une activité d'effecteur in-vivo d'un polypeptide avec la séquence de la Figure 4 et n'ayant pas la glycosylation naturelle de la protéine porteuse.

2. Polypeptide selon la revendication 1 avec la séquence de la Figure 4.

3. Polypeptide selon la revendication 1 ou la revendication 2 qui est substantiellement exempt de substances naturellement présentes dans le sérum humain.

4. Polypeptide essentiellement pur ayant la séquence des acides aminés 27 à 290 de la Figure 4 et ayant un résidu méthionine précédant l'acide aminé 27.

5. Polypeptide selon la revendication 4, dans lequel un ou plus résidus acides aminés ont été ajoutés, effacés ou substitués et le polypeptide constitue un polypeptide analogue à une protéine porteuse.

6. Polypeptide choisi dans le groupe consistant en un polypeptide ayant la séquence des acides aminés -1 à 290 de la Figure 4, et polypeptides ayant une portion de cette séquence et constituant un polypeptide analogue à une protéine porteuse.

7. Polypeptide selon l'une quelconque des revendications 1 à 6 substantiellement complexé avec au moins un polypeptide analogue à la somatomédine.

8. Procédé qui comprend la préparation d'une composition thérapeutique qui comprend une quantité efficace d'un polypeptide tel que défini dans l'une quelconque des revendications 1 à 6 pour inhiber la croissance des cicatrices chéloïdiennes et pour inhiber la croissance des cancers dépendant de la somatomédine.

9. Procédé qui comprend la préparation d'une composition thérapeutique qui comprend une quantité efficace d'un polypeptide tel que défini dans l'une quelconque des revendications 1 à 6 complexé substantiellement avec au moins un polypeptide analogue à la somatomédine pour traiter l'ostéoporose chez les humains.

10. Utilisation d'un polypeptide selon l'une quelconque des revendications 1 à 6 dans la fabrication d'un médicament pour inhiber la croissance des cicatrices chéloïdiennes ou dans la fabrication de médicaments pour inhiber la croissance des cancers dépendant de la somatomédine.

11. Utilisation d'un polypeptide selon la revendication 6 dans la fabrication d'un médicament pour le traitement de l'ostéoporose.

12. Isolat d'ADN qui comprend une séquence d'ADN qui code pour un polypeptide ayant la séquence des acides aminés -1 à 290 de la Figure 4.

13. Isolat d'ADN qui comprend une séquence d'ADN qui code pour un polypeptide ayant la séquence des acides aminés 27 à 290 de la Figure 4.

14. Isolat d'ADN qui comprend une séquence d'ADN qui code pour un polypeptide ayant la séquence des acides aminés 27 à 290 de la Figure 4 et ayant un résidu méthionine précédant l'acide aminé 27.

15. Molécule d'ADN recombinant qui comprend une séquence d'ADN telle que définie dans l'une quelconque des revendications 12 à 14 pontée opérationnellement à une séquence de contrôle de l'expression.

16. Cellule hôte transformée avec la molécule d'ADN recombinant selon la revendication 15.

17. Méthode pour produire un polypeptide analogue à une protéine porteuse, comprenant la culture d'une cellule hôte selon la revendication 16 pour fabriquer ledit polypeptide.

18. Vecteur comprenant une molécule d'ADN recombinant selon la revendication 15.

19. Hôte transformé avec un vecteur selon la revendication 18.

20. Méthode pour produire un polypeptide analogue à une protéine porteuse qui comprend la culture d'un hôte transformé par un vecteur selon la revendication 18 pour fabriquer ledit polypeptide.
